(19) **Europäisches Patentamt / European Patent Office / Office européen des brevets**

(11) **EP 4 527 389 A1**

(12) **EUROPEAN PATENT APPLICATION**
published in accordance with Art. 153(4) EPC

(43) Date of publication:
26.03.2025 Bulletin 2025/13

(21) Application number: 23815349.8

(22) Date of filing: 18.05.2023

(51) International Patent Classification (IPC):
*A61K 31/59* (2006.01)    *A61K 47/20* (2006.01)
*A61K 47/34* (2017.01)    *A61K 9/10* (2006.01)
*A61P 25/18* (2006.01)

(52) Cooperative Patent Classification (CPC):
A61K 9/10; A61K 31/59; A61K 47/20; A61K 47/34;
A61P 25/18

(86) International application number:
PCT/ES2023/070320

(87) International publication number:
WO 2023/233051 (07.12.2023 Gazette 2023/49)

(84) Designated Contracting States:
AL AT BE BG CH CY CZ DE DK EE ES FI FR GB
GR HR HU IE IS IT LI LT LU LV MC ME MK MT NL
NO PL PT RO RS SE SI SK SM TR
Designated Extension States:
BA
Designated Validation States:
KH MA MD TN

(30) Priority: 18.05.2022  US 202263343221 P

(71) Applicant: Laboratorios Farmacéuticos Rovi S.A.
28037 Madrid (ES)

(72) Inventors:
• GUTIERRO ADURIZ, Ibon
  Madrid (ES)
• FRANCO RODRIGUEZ, Guillermo
  Madrid (ES)

(74) Representative: Elzaburu S.L.P.
Paseo de la Castellana 259C
Torre de Cristal, planta 28
28046 Madrid (ES)

(54) **PROLONGED-RELEASE INJECTABLE COMPOSITIONS FOR USE IN TREATMENT WITH RISPERIDONE TOGETHER WITH CYP2D6 ENZYME INHIBITORS**

(57)    The invention relates to prolonged-release injectable compositions for use in a method for treating a disease, disorder or condition responsive to risperidone or to paliperidone with risperidone in a subject who receives one or more inhibitors of the enzyme CYP2D6 (cytochrome P450 2D6). The invention also relates to prolonged-release injectable compositions for use in a method for treating a disease, disorder or condition responsive to risperidone or to paliperidone with risperidone and for simultaneously treating a disease, disorder or condition responsive to CYP2D6 enzyme inhibitor in a subject. The invention further relates to prolonged-release injectable compositions containing risperidone and a CYP2D6 enzyme inhibitor, wherein the dose of risperidone does not need to be reduced according to the administered dose of CYP2D6 inhibitor.

EP 4 527 389 A1

**Description**

**FIELD OF THE INVENTION**

The present invention relates to compositions for use in a method for treating a disease, disorder or condition responsive to risperidone or paliperidone with risperidone in a subject receiving one or more other therapeutic agents that are CYP2D6 (cytochrome P450 2D6) enzyme inhibitors. In particular, the method uses administration of risperidone and one or more other therapeutic agents in the same or separate dosage forms. The risperidone is included in a long-acting injectable (LAI) depot composition (prolonged-release injectable suspension) comprising risperidone for treating a disease, disorder or condition responsive to risperidone or responsive to paliperidone. The CYP2D6 enzyme inhibitor(s) may be used to treat one or more disorders.

The method provides for intramuscular administration of the prolonged-release injectable composition about once every 28 days (or about once monthly), does not require prior administration of risperidone loading dose(s) of said prolonged-release injectable composition, and does not require concomitant administration of the prolonged-release injectable composition and oral supplementation with risperidone. The composition provides a therapeutically effective plasma concentration of risperidone from the first day of administration (within 8 to 24 hours after administration) throughout a period of at least about 28-31 days or more. The composition provides improved performance over other LAI depot compositions. For patients receiving the one or more other therapeutic agents, the dose of risperidone in the prolonged-release injectable composition is preselected to reduce the impact of the other therapeutic agent(s) on the plasma concentration of risperidone and/or its metabolite (9-hydroxyl-risperidone; paliperidone).

**BACKGROUND TO THE INVENTION**

Patients with schizophrenia or bipolar disorder are often treated with risperidone. Those patients, however, also often suffer from depression, which may be treated by administration of a selective serotonin reuptake inhibitor (SSRI) antidepressant.

PAXIL (paroxetine hydrochloride) and PROZAC (fluoxetine) are SSRI antidepressants used to treat depression, panic attacks, obsessive-compulsive disorder (OCD), and a severe form of premenstrual syndrome (premenstrual dysphoric disorder). PAXIL is also used to treat anxiety disorders (social and/or general anxiety) and post-traumatic stress disorder. PROZAC is also used to treat bulimia nervosa, major depressive disorder, panic disorder, depressive disorders associated with bipolar I disorder (when taken with olanzapine: ZYPREXA), and treatment-resistant depression (depression that has not improved with at least two other treatments) taken with olanzapine (ZYPREXA).

Risperidone and fluoxetine are strong CYP2D6 inhibitors. Risperidone is mainly metabolised to 9-hydroxyl-risperidone (paliperidone) by CYP2D6, so administration of risperidone and an SSRI to a subject can cause undesired changes in the plasma concentration of risperidone. Neither the package leaflet for PROZAC nor the package leaflet for PAXIL provides guidance on how administration of PROZAC or PAXIL should be altered when administered to a subject receiving a prolonged-release injectable composition containing risperidone, in particular, the prolonged-release injectable composition of the invention as described herein. Daily oral dosing of risperidone provides a different overall plasma concentration profile than monthly dosing of risperidone in a prolonged-release injectable composition. Accordingly, it is not possible to use the recommended daily oral dosing regimen of risperidone as a guide to determine a suitable monthly intramuscular dosing regimen of risperidone in a prolonged-release injectable composition.

Schizophrenia may occur in various levels of intensity. Many patients with these mental illnesses achieve symptom stability with available oral anti-psychotic medications; however, it is estimated that up to 75% have difficulty adhering to a daily oral treatment regimen, i.e. compliance problems. Problems with adherence often result in worsening of symptoms, suboptimal treatment response, frequent relapses and re-hospitalisations, and an inability to benefit from rehabilitative and psychosocial therapies.

Risperidone, and its primary active metabolite 9-OH-risperidone (paliperidone), are indicated for the treatment of psychotic disorders such as schizophrenia, schizoaffective disorder, bipolar disorder, and bipolar mania. Risperidone can be administered orally in commercially available tablet, solution, or orally disintegrating tablet dosage forms.

Numerous publications discuss the proper use of oral risperidone versus La Is containing risperidone for the treatment of the various degrees of schizophrenia. Citrome (Clinical Schizo. Related Psych., (2018), 130-141), Bai et al. (Pharmacopsych. (2006), 39, 135-141), and Andorn et al. (J. Clin. Psychopharm., (2019), 39(5), 428-433). Patients with schizophrenia suffering a relapse need urgent attention owing to the severity of the symptomatology and future consequences if not treated immediately.

Risperidone is suitable for treating schizophrenia and bipolar disorder, among other psychotic disorders. Risperidone is available in rapid (immediate) release oral and prolonged-release injectable dosage forms.

LAI depot compositions are known: US 8,221,778 to Siegel et al. (corresponding to WO 2005/070332), US 5,688,801, US 6,803,055, US 5,770,231, US 7,118,763, US 4,389,330 to Dunn, US 4,530,840, US 6,673,767 to Brodebeck, US 6,143,314 to Chandrashekar, WO 2004/081196, WO 2001/035929, WO 2008/153611 A2 to QLT USA, WO 2000/024374, WO 2002/038185, WO 2008/100576, WO 2011/151355 A1 to Laboratorios Farmaceuticos Rovi, S.A., WO 2011/42453, US 10085936 to Gutierro Aduriz, US 10463607 to Gutierro Aduriz, US 10182982 to Gutierro Aduriz, US 2020/0085728 A1 to Gutierro Aduriz, EP 2394664 A1 to Laboratorios Farmaceuticos ROVI, S.A., US WO 2011/151355 A1 to Laboratorios Farmaceuticos ROVI, S.A., US 10058504 Gutierro Aduriz, US 10881605 Gutierro Aduriz, US 10195138 Gutierro Aduriz, US 2021/0077380 A1 to Laboratorios Farmaceuticos ROVI, S.A., EP 2394663 A1 to Laboratorios Farmaceuticos ROVI, S.A., WO 2011/151356 A2 to Laboratorios Farmaceuticos ROVI, S.A., US 10350159 to Gutierro Aduriz, US 2019/0328654 A1 to Laboratorios Farmaceuticos ROVI, S.A., EP 2529757 A1 to Laboratorios Farmaceuticos ROVI, S.A., WO 2013/178811 A1 to Laboratorios Farmaceuticos ROVI, S.A., US 10335366 to Gutierro Aduriz, US 2019/0254960 A1 to Laboratorios Farmaceuticos ROVI, S.A., US 11007139 to Gutierro Aduriz, EP 2529756 A2 to Laboratorios Farmaceuticos ROVI, S.A., WO 2013/178812 A1 to Laboratorios Farmaceuticos ROVI, S.A., US 2008/0287464 A1 to Wright, US 2009/0264491 A1 to McKay, US 2004/0010224 A1 to Bodmeier, US 2007/0077304 A1 to Luk, US 2010/0015195 A1 to Jain, US 2010/0266655 A1 to Dadey, WO 95/29664 to Alkermes, WO 2004/011054 A2 to Alza, WO 2007/041410 A2 to Luk, WO 2008/059058 A1 to Bourges, WO 2010/018159 A1 to Schoenhammer.

Two such LAI products containing risperidone have been approved by the U.S.F.D.A.

RISPERDAL Consta® (NDA N021346; dosage strengths- 12.5 mg/vial, 25 mg/vial, 37.5 mg/vial and 50 mg/vial; US 6596316, US 6379703, US 6194006, WO 2000/40221) is an intramuscular risperidone-containing PLGA microparticle formulation, and it is intended to deliver therapeutic levels of risperidone suitable for bi-weekly administration. However, due to the inherent lag phase of most microparticle-based products, the patient is required to supplement the first weeks with daily doses of oral risperidone after first administration. Approximately three weeks after a single intramuscular injection of Risperdal Consta® and concurrent daily doses of oral risperidone, the microspheres release sufficient risperidone in the systemic circulation that the patient can discontinue supplementation with daily doses of the oral therapy. However, this period of oral supplementation could be a risk factor of therapeutic non-compliance. Furthermore, the presence in the body of two doses at the same time could present a potential risk of adverse events, such as irregular formulation behaviour and toxicity.

PERSERIS® (NDA N210655; dosage strengths- 90 mg and 120 mg per dose; US 9180197, US 9186413, US 9597402, US 10010612, US 10058554, US 10376590, US 10406160) is a depot formulation containing risperidone in microparticles intended for subcutaneous administration in adipose (fatty) tissue. Nasser et al. ("Efficacy, safety and tolerability of RBP-7000 once-monthly risperidone for the treatment of acute schizophrenia: an 8-week, randomized, double-blind, placebo-controlled, multicenter Phase 3 study" in J. Clin. Psycopharm. (2016), 36(2), 130-140) evaluated the RBP-7000 LAI (90 mg and 120 mg dosage strengths) for treating acute schizophrenia.

Another LAI product containing risperidone is still undergoing clinical evaluation for the treatment of schizophrenia: Correll et al. (NPJ Schizophrenia (Nov. 25, 2020), 6:37), Edison Investment Research Limited ("Doria Phase III Trial hits primary endpoint, Laboratorios Farmaceuticos ROVI, S.A. March 19, 2019, www.edisongroup.com/publication/doria-phase-iii-trial-hits-primary-endpoint/23705/), NCT02086786, NCT03160521, NCT01788774, NCT01320410, NCT03870880, NCT03160521, NCT01788774, and Anta et al. (Newer formulations of risperidone: remarks about Risperidone ISM" in CNS Drugs (Sep. 52020). The composition of the product is not disclosed in those publications. Risperidone-containing LAI depot compositions interact differently with other drugs, because said depot compositions differ according to form of administration, dose of risperidone administered, formulation of the depot composition, and/or the pharmacokinetics provided by the depot composition. For example, the specifics of drug-drug interactions of PERSERIS would be different than those of RISPERDAL CONSTA and would be different than that of the prolonged-release injectable composition used according to the invention herein below.

The U.S. document 6,331,311 issued to Brodbeck also discloses injectable depot compositions comprising a biocompatible polymer such as PLGA, a solvent such as N-methyl-2-pyrrolidone and a beneficial agent such as a drug, further comprising an emulsifying agent such as polyols. However, the compositions disclosed do not perform satisfactorily when the beneficial agent is risperidone because the use of a two-phase composition with emulsifying agents accelerates implant hydration and increases effective releasing surface area, impairing the control on the initial burst release and originating a fast decrease in drug release from the first days to the following ones. For example, a comparison composition was prepared according to patent '311. A container containing risperidone (150 mg), PLGA (300 mg, having an inherent viscosity of 0.32 dl/g and irradiated by β-irradiation at a dose of 25 KGy) and NMP (700 mg) was prepared. Another container contained polyvinyl alcohol in water (1 ml of 2% w/v). The contents of the containers were mixed, then the mixture was transferred to a syringe and injected intramuscularly (an amount equivalent to 2.5 mg risperidone) into the gluteus of New Zealand white rabbits (n=3). More than 70% of the total AUC of active moiety was released within the first 5 days after the injection. Such a formulation is unable to provide therapeutic plasma levels of risperidone for a period of at least four weeks.

U.S. 4,938,763, issued to Dunn et al., discloses a method for an injectable in situ forming implant. A biodegradable polymer or copolymer dissolved in a water-miscible solvent with a biologically active agent is either dissolved or dispersed within the polymeric solution. Once the polymeric solution is exposed to body fluids, the solvent diffuses and the polymer solidifies thereby entrapping the drug within the polymer matrix. Although Dunn et al. discloses the use of water-miscible solvents for obtaining in situ forming polymeric implants, it discloses a number of polymers and solvents and even proportions between the different ingredients that do not produce a satisfactory implant with the appropriate release characteristics, particularly when the implant contains risperidone as active principle. For example, a comparison composition was prepared according to patent '763. A container containing risperidone (50 mg) and PLGA (784 mg, monomer ratio of lactic acid to glycolic acid monomer of 75:25, and having an inherent viscosity of 0,20 dl/g) was prepared. Another container containing NMP (1666 mg) was prepared. The contents of the containers were mixed. Then the mixture was transferred to a syringe and a portion (1250 mg, corresponding to 25 mg of risperidone) was injected into an aqueous liquid to determine its *in vitro* release profile. More than 50% of the risperidone was released within the first 2 days. Such a formulation is unable to provide therapeutic plasma levels of risperidone for a period of at least four weeks.

A subject being treated with a CYP2D6 enzyme inhibitor and oral risperidone must decrease the dose of oral risperidone due to the impact that the inhibitor has on plasma concentration of risperidone. For example, the package leaflet for PAXIL (paroxetine hydrochloride) indicates that the initial dose and the maintenance dose vary according to the condition being treated. It also states: "Concomitant use of paroxetine with risperidone, a CYP2D6 substrate has also been evaluated. In one study, daily dosing of paroxetine 20 mg in patients stabilized on risperidone (4 to 8 mg/day) increased mean plasma concentrations of risperidone approximately 4-fold, decreased 9-hydroxyrisperidone concentrations approximately 10%, and increased concentrations of the active moiety (the sum of risperidone plus 9-hydroxyrisperidone) approximately 1.4-fold." As a result, the daily dose of oral risperidone must be adjusted (reduced, not maintained) based upon the dose of CYP2D6 being administered.

The art in the field of LAI depot compositions does not disclose the specific dose combinations that should be used when the prolonged-release injectable composition of the invention is administered to a subject also receiving CYP2D6 inhibitor, such as a SSRI antidepressant, e.g. paroxetine or fluoxetine. Given the increasing popularity of the risperidone-containing prolonged-release injectable composition, it would be a significant advance in the art to provide a method of treating patients with both a risperidone-containing prolonged-release injectable composition and a SSRI antidepressant, wherein the dose of risperidone does not have to be adjusted based upon the dose of SSRI administered.

A need also remains in the art for improved methods of treating episodes of schizophrenia, whether first time or recurring, in particular of acute exacerbation of schizophrenia. It would be a significant advance in the art to provide an improved method of treating acute exacerbation of schizophrenia with a prolonged-release injectable composition comprising risperidone as first line therapy, wherein the method would not require oral risperidone supplementation or risperidone loading dose(s) of said prolonged-release injectable composition, and wherein the composition would provide therapeutically effective plasma levels of risperidone within 8 to 24 hours after administration and would provide efficacious treatment of the acute episode within about 8 days or within about 15 days after administration and wherein the psychotic patient is also receiving a SSRI antidepressant. It would be a further improvement in the art to provide a method requiring a reduced dose of risperidone in a prolonged-release injectable composition for treating acute exacerbation of schizophrenia, because a reduced dose would result in a lower incidence of drug-related adverse events (side effects) as compared to other known LAI depot compositions of risperidone on a dose equivalent basis, and it would reduce the level of interaction with the SSRI antidepressant.

## SUMMARY OF THE INVENTION

The present invention seeks to provide an improved composition for use in a method of treating a disease, disorder or condition responsive to risperidone or responsive to paliperidone in a subject also being treated with (administered) CYP2D6 (cytochrome P450 2D6) enzyme inhibitor, e.g. SSRI antidepressant. In some embodiments, the subject is being treated for depression and a disease, disorder or condition responsive to risperidone.

The present invention includes injectable depot composition(s), implant(s) formed from said injectable depot composition(s), method(s) of forming (preparing) said implant(s), kit(s) comprising components used to form said prolonged-release injectable composition(s), method(s) of preparing said injectable depot compositions, method(s) of administering risperidone by administering said prolonged-release injectable composition(s), method(s) of treating disease(s), condition(s), or disorder(s) that is/are therapeutically responsive to risperidone and/or paliperidone (9-hydroxyrisperidone), and method(s) of treating disease(s), condition(s), or disorder(s) that is/are therapeutically responsive to CYP2D6 enzyme inhibitor by administering said prolonged-release injectable composition(s) and said CYP2D6 enzyme inhibitor. In particular, references to therapeutic, surgical or diagnostic methods of treatment herein are to be interpreted as references to the compounds, pharmaceutical compositions and drugs for use in such methods.

After administration, the prolonged-release injectable composition provides therapeutic plasma levels of active moiety from the start and continuously over a period of at least about 28 days. It does not require oral supplementation with risperidone or paliperidone nor does it require loading doses of oral risperidone or oral paliperidone to achieve the target steady state plasma concentration of active moiety. Unlike the use of other LAI depot (long-acting injectable) compositions containing risperidone, the method of the invention also provides the advantage that therapeutic plasma concentrations of active moiety can be maintained following termination of an oral dosing regimen of risperidone by administering the prolonged-release injectable composition as defined herein. Furthermore, unlike the use of other LAI depot compositions containing risperidone, the method of the invention also provides the advantage that therapeutic plasma concentrations of active moiety can be maintained following termination of a prolonged-release injectable composition dosing regimen by orally administering once-daily doses of risperidone. Therapeutic levels of active moiety are provided from the first day to the last day of a dosing period.

As compared to the use of other LAI depot compositions containing risperidone, the method of the invention a) more rapidly achieves and maintains plasma concentration of active moiety (of risperidone and its metabolite 9-OH-risperidone, if present) in the therapeutic range; and b) provides a substantially improved clinical benefit (therapeutic result). Unlike the use of other LAI depot compositions containing risperidone, the method of the invention also provides the advantage that therapeutic plasma concentrations of active moiety can be maintained following termination of an oral dosing regimen of risperidone by administering the prolonged-release injectable composition as defined herein.

The invention uses a dosing regimen of intramuscular administration of about 75-100 mg, about 25-75 mg, or about 75 mg of risperidone (in a prolonged-release injectable composition as described herein) about once every 28 days. It does not require oral supplementation with risperidone as RISPERDAL CONSTA does (as described in NDA N021346). It provides a therapeutically effective plasma concentration of risperidone from the first day of administration (within 2 to 24 hours or within 8 to 24 hours after administration) throughout a period of about 27-31 days, about 28-31 days, or about 27-29 days, unlike Risperdal Consta and PERSERIS (as described in NDA N210655). Also, unlike the use of other LAI depot compositions containing risperidone, the method of the invention also provides the advantage that therapeutic plasma concentrations of active moiety (risperidone and/or paliperidone) can be maintained following termination of a prolonged-release injectable composition dosing regimen by orally administering once-daily doses of risperidone.

The prolonged-release injectable composition may be administered to a subject already undergoing treatment with a CYP2D6 (cytochrome P450 2D6) enzyme inhibitor. The invention provides a method of treating a disease, disorder or condition responsive to risperidone in a subject also undergoing therapy with a drug that is a CYP2D6 enzyme inhibitor, the method comprising administering to said subject via intramuscular injection a prolonged-release injectable composition comprising about 75 mg of risperidone, DMSO and PLGA copolymer. In preferred embodiments, the dose of said prolonged-release injectable composition is maintained (about the same throughout a treatment period) after administration of said CYP2D6 inhibitor, and a dose of said prolonged-release injectable composition is administered at least about every four weeks.

Administration of the CYP2D6 enzyme inhibitor to the subject may also begin after administration of the prolonged-release injectable composition. The invention thus provides a method of treating both a disease, disorder or condition responsive to risperidone in a subject and a disease, disorder or condition responsive to CYP2D6 enzyme inhibitor in the subject, the method comprising a) administering to said subject via intramuscular injection a dose of long-acting injectable (LAI) depot composition comprising about 75 mg of risperidone, DMSO and PLGA copolymer; and b) within 2 to 4 weeks of said administration, administering to said subject a dose of said CYP2D6 enzyme inhibitor. In preferred embodiments, the dose of said prolonged-release injectable composition is maintained (about the same) throughout a treatment period after administration of said CYP2D6 enzyme inhibitor, and a dose of said prolonged-release injectable composition is administered about every four weeks.

The invention also provides a method of switching a subject, undergoing treatment with oral risperidone and a CYP2D6 enzyme inhibitor, from a once-daily oral dosing regimen of risperidone to a once-every 28 days prolonged-release injectable composition dosing regimen of risperidone, the method comprising a) discontinuing once-daily oral administration of risperidone in a subject in need thereof; and b) within 24-48 hours or within 24 hours of said discontinuation, administering to said subject a prolonged-release injectable composition as disclosed herein comprising a dose of about 75 mg of risperidone, thereby maintaining a therapeutically effective plasma concentration of active moiety in said subject for about 28 days. In some embodiments, wherein the subject has been receiving once-daily oral doses of less than 4 mg of risperidone, the subject is administered an amount of prolonged-release injectable composition, as described herein, comprising up to (or no more than) about 75 mg of risperidone. In other embodiments, wherein the subject has been receiving once-daily oral doses of 4 mg or more of risperidone and/or up to 6 mg of risperidone, the subject is administered an amount of prolonged-release injectable composition, as described herein, comprising no more than about 75 mg of risperidone. In some embodiments, the dose of CYP2D6 is not altered due to start of treatment with the prolonged-release injectable composition.

The invention also provides a method of switching a subject, undergoing treatment with oral risperidone and a CYP2D6 enzyme inhibitor, from an oral risperidone dosing protocol to a long-acting injectable (LAI) composition risperidone dosing protocol, the method comprising a) identifying a subject being administered oral risperidone; b) discontinuing said oral risperidone; and c) about every four weeks or about once-monthly intramuscularly administering to said subject an amount of prolonged-release injectable composition comprising a dose of about 75 mg of risperidone. In some embodiments, the first dose of the prolonged-release injectable composition is administered within 24 to 48 h or within 24 h after said discontinuation. The dose of oral risperidone being administered to the subject may be less than about 4 mg daily, or it may be about 4 mg daily up to about 6 mg daily or up to about 8 mg daily. In some embodiments, the dose of CYP2D6 is not altered due to discontinuation of treatment with the prolonged-release injectable composition.

The CYP2D6 enzyme inhibitor may be administered in one or more separate dosage forms. In some embodiments, the CYP2D6 enzyme inhibitor is a selective serotonin reuptake inhibitor (SSRI) antidepressant. The SSRI may be selected from the group consisting of paroxetine (or salt thereof) and fluoxetine (or salt thereof). A subject being administered the SSRI may be undergoing treatment for depression, panic attacks, obsessive-compulsive disorder (OCD), a severe form of premenstrual syndrome (premenstrual dysphoric disorder), anxiety disorders (social and/or general anxiety), post-traumatic stress disorder, bulimia nervosa, major depressive disorder, panic disorder, depressive disorders associated with bipolar I disorder, or treatment-resistant depression (depression that has not improved with at least two other treatments).

The invention provides suitable dosing regimens for a subject being administered a CYP2D6 enzyme inhibitor, such as a SSRI and the risperidone-containing prolonged-release injectable composition. Example CYP2D6 enzyme inhibitors that can be administered to the subject being administered the risperidone-containing prolonged-release injectable composition are listed below.

| CYP2D6: | Strong inhibitors | Moderate inhibitors | Weak inhibitors |
|---|---|---|---|
| | bupropion, fluoxetine, paroxetine, quinidine, terbinafine | abiraterone, cinacalcet, duloxetine, lorcaserin, mirabegron | amiodarone, celecoxib, cimetidine, clobazam, cobicistat, escitalopram, fluvoxamine, labetalol, ritonavir, sertraline, vemurafenib |

The invention provides a method of treating a mental disorder responsive to risperidone or responsive to paliperidone in a subject in need thereof, the method comprising administering to said subject via intramuscular injection a dose of prolonged-release injectable composition, as described herein, wherein said subject is also undergoing therapy with a SSRI antidepressant, and said dose of prolonged-release injectable composition comprises about 75 mg of risperidone. In some embodiments, the SSRI antidepressant is PEG2000 (or PEG-2K).

Aspects of the invention includes those wherein the initial dose of the prolonged-release injectable composition is the same strength as the maintenance dose of said prolonged-release injectable composition. For example, for a subject receiving an initial dose of about 75 mg of risperidone in the prolonged-release injectable composition, the second and subsequent maintenance doses of said composition will comprise about 75 mg of dose of risperidone. Accordingly, the invention includes embodiments wherein the dosage strength of risperidone in prolonged-release injectable composition doses administered to a subject remain the substantially the same throughout a treatment period during which a subject is also undergoing treatment with CYP2D6 enzyme inhibitor.

A preferred embodiment of the invention provides a method of treating a disease, disorder or condition responsive to risperidone or responsive to paliperidone and a disease, disorder or condition responsive to SSRI in a subject, the method comprising, in no particular order, a) administering to the subject a risperidone-containing prolonged-release injectable composition as defined herein comprising a dose of no more than about 75 mg of risperidone; and b) administering to the subject a therapeutically effective dose of SSRI.

In some embodiments, prior to being administered the prolonged-release injectable composition, the subject a) is unstable and experiencing severe to moderate psychotic symptoms; b) is experiencing a first acute exacerbation of schizophrenia; c) is undergoing treatment with one or more oral antipsychotic drugs; d) has experienced prior episode(s) of acute exacerbation of schizophrenia; e) is experiencing worsening psychotic symptoms or impending relapse of psychosis; f) is experiencing a relapse of severe to moderate psychotic symptoms; and/or g) is undergoing treatment with a prolonged-release injectable composition not according to the invention.

In some embodiments, a) the prolonged-release injectable composition comprises about 75 mg of risperidone, DMSO, and PLGA copolymer; b) the prolonged-release injectable composition forms a biodegradable implant in muscle after administration; c) ≤2.5%, ≤ 5%, ≤ 7.5%, ≤ 10%, ≤20% of the risperidone is dissolved in said prolonged-release injectable composition before administration; d) >0%, ≥0.5%, ≥1%, ≥5%, ≥10%, ≥15%, or up to about 20% wt of the risperidone is dissolved in said composition before administration; e) the PLGA copolymer has a monomer ratio of lactic acid to glycolic acid in the range from about 50:50 to about 75:25, about 35:65 to about 75:25, about 45:55 to about 70:30, about 50:50 to about 65:35, or about 65:35 to about 75:25, 45:55 to 55:45, or 48:52 to 52:48, or about 50:50, i.e. 50:50 ±10%, or 75:25 ±10%; f) before administration, a polymer solution used to form the prolonged-release injectable composition has a viscosity in the range of about 0.5-7 Pa·s, about 0.5-4. Pa·s, about 0.7-4 Pa·s, about 0.5-3.0 Pa·s, about 0.7-3.0 Pa·s, about 1.5-2.1 Pa·s ±10%, about 1.5 to about 2.5 Pa·s, about 1.5 to about 2.3 Pa·s, or about 1.7 - 1.8 Pa·s ±10%; g) the PLGA copolymer has an inherent viscosity in the range of 0.20-0.60 dl/g, about 0.30-0.55 dl/g, about 0.36-0.52 dl/g, about 0.40-0.58 dl/g, or about 0.46-0.51 dl/g measured in chloroform at 30°C and at a concentration of 0.5% wt. with a size 0B Ubbelohde glass capillary viscometer; h) the prolonged-release injectable composition has a mass ratio of DMSO to risperidone from about 5:1 to about 4:1, from about 4.6:1 to about 4.8:1, from about 4.6:1 to about 4.7:1, about 4.67:1, about 4.66:1 or about 4.68:1, or about 4.66:1; i) the prolonged-release injectable composition has a mass ratio of risperidone to (PLGA + risperidone), expressed as the percentage of the weight of risperidone with relative to the total weight of the risperidone plus PLGA, in the range of about 15-40% wt, about 25-35% wt, about 30-35%, about 31-35%, about 32-34% or about 33% wt; j) the PLGA copolymer is end-capped with an ester group or a carboxyl group; k) the content of risperidone in the formulation is about 10-15% wt, about 11-14% wt, about 12-14% wt or about 13% wt; l) the risperidone is partially dissolved or substantially not completely dissolved in said composition; m) the PLGA polymer has been irradiated with beta or gamma radiation, preferably in the range of 10-30 KGy, more preferably in the range of 15-30 Kgy, and most preferably between 16-25 Kgy $\pm$10%; n) the prolonged-release injectable composition is sterile; o) the injectable composition continuously provides therapeutically effective plasma levels of drug in the subject over a dosing period of at least four weeks from the day of administration; o) the injectable composition continuously provides therapeutically effective plasma levels of drug in the subject over a dosing period of at least four weeks from the day of administration; p) prior to administration, the prolonged-release injectable composition has a viscosity in the range of about 1.0-7.0 Pa·s, about 1.5-7.0 Pa·s, or about 1.8-6.5 Pa·s; q) the mass ratio of solvent (DMSO) to a polymer solution, expressed as the weight percentage of solvent with respect to the weight of polymer + solvent, is about 50-75%, about 65-75%, about 60-70%, about 68-72%, or about 70%; r) the concentration of PLGA in the prolonged-release injectable composition is in the range of 24-50% wt, 24-40% wt, 24-30% wt, 25-27% wt, or 26% wt, (expressed as the weight percentage of polymer based on the total weight of the composition); and/or s) the DMSO content in the prolonged-release injectable composition is about 55-65% wt, about 57-63% wt, about 60-62% wt, or about 61% wt based on the total weight of the prolonged-release injectable composition.

For a 75 mg drug dosage strength, an example pharmaceutical kit may comprise about 90 mg (or about 75-105 mg or about 80-100 mg or about 85-105 mg or about 85-95 mg) of drug (which is risperidone, paliperidone, or a mixture thereof), about 420 mg (or about 405-435 mg or about 415-425 mg) of DMSO, and about 180 mg (or about 165-200 mg or about 170-190 mg or about 175-185 mg) of PLGA having a L:G monomer molar ratio from about 45:55 to about 55:45 (or about 50:50).

In some embodiments, prior to administration, the prolonged-release injectable composition comprises about 75 mg (or about 65-85 mg or about 70-80 mg) of drug, DMSO, and PLGA, wherein the drug content is about 10-15% wt, the DMSO content is about 55-65% wt, the PLGA content is about 24%-30% wt, and the PLGA has a L:G monomer ratio from about 45:55 to 55:45.

In some embodiments, prior to administration, the prolonged-release injectable composition comprises about 75 mg (or about 65-85 mg or about 70-80 mg) of drug, DMSO, and PLGA, wherein the drug content is about 12-14% wt, the DMSO content is about 57-63% wt, the PLGA content is about 25-27% wt, and the PLGA has a L:G monomer ratio from about 45:55 to 55:45.

For a 100 mg drug dosage strength, an example pharmaceutical kit may comprise about 115 mg (or about 100-130 mg or about 105-125 mg or about 110-120 mg) of drug (which is risperidone, paliperidone, or a mixture thereof), about 537 mg (or about 515-560 mg or about 520-550 mg or about 530-545 mg) of DMSO, and about 230 mg (or about 215-245 mg or about 220 to about 235 mg) of PLGA having a L:G monomer molar ratio from about 45:55 to about 55:45 (or about 50:50).

In some embodiments, prior to administration, the prolonged-release injectable composition comprises about 100 mg (or about 85-115 mg or about 90-110 mg or about 95-105 mg) of drug, DMSO, and PLGA, wherein the drug content is about 10-15% wt, the DMSO content is about 55-65% wt, the PLGA content is about 24%-30% wt, and the PLGA has a L:G monomer ratio from about 45:55 to 55:45.

In some embodiments, prior to administration, the prolonged-release injectable composition comprises about 100 mg (or about 85-115 mg or about 90-110 mg or about 95-105 mg) of drug, DMSO, and PLGA, wherein the drug content is about 12-14% wt, the DMSO content is about 57-63% wt, the PLGA content is about 25-27% wt, and the PLGA has a L:G monomer ratio from about 45:55 to 55:45.

In some embodiments, the method of preparing the prolonged-release injectable composition comprises a) mixing DMSO with a powdered mixture of PLGA and drug for a period of 10 minutes or less, 5 minutes or less, 4 minutes or less, 3 minutes or less, 2 minutes or less, 1 minute or less, or 30 seconds or less, thereby forming said prolonged-release injectable composition, wherein at least 80% wt of the drug is suspended therein and wherein the PLGA is completely dissolved therein. When using syringes as the containers, mixing of the components is accomplished by engaging the syringes with each other and pushing-pulling their respective plungers repeatedly as needed. In some embodiments, the plungers are pushed through 200 pushes (runs) or less, 150 pushes or less, 100 pushes or less, 75 pushes or less, or 50 pushes. A push relates to passage of the liquid from one syringe to another.

The prolonged-release injectable composition may be administered intramuscularly or subcutaneously. It may also be administered into adipose tissue. Intramuscular administration into the gluteal or deltoid muscle is preferred.

In some embodiments, the method a) excludes the step of administering one or more (plural) oral loading doses of risperidone in said prolonged-release injectable composition before said intramuscular administration of the prolonged-release injectable composition; and/or b) excludes the step of orally administering one or more doses of risperidone within said 28-day or monthly period.

The composition used in the method of the invention may be provided as a pharmaceutical kit that forms said composition. In some embodiments, the drug and the biocompatible polymer are contained in a first container, and the DMSO is contained in a second, separate container. Preferably, at least one of the first and second containers is a syringe, a vial, a device or a cartridge, either disposable or not and more preferably both the first and the second containers are disposable syringes. The containers can be syringes, vials, capsules, ampoules, devices or cartridges. In another embodiment, each of the three ingredients is contained in its own container. In another embodiment, the DMSO and risperidone are contained in a first container and the polymer is contained in a second container. When required, the contents of both containers are combined, for example by means of a connector or by means of male-female syringes, and mixed together so that the compositions according to the invention are reconstituted, for example by moving the plungers of the syringes forwards and backwards.

Accordingly, the method of treatment can further comprise the step(s) of a) providing a pharmaceutical kit comprising at least two containers within which the ingredients of the prolonged-release injectable composition are divided, and mixing the contents of said containers to form said composition; b) providing a pharmaceutical kit comprising at least two containers within which the ingredients of the prolonged-release injectable composition are divided; and b) providing a pharmaceutical kit comprising at least two containers within which the ingredients of the prolonged-release injectable composition are divided and mixing the contents of a first container with the contents of a second container to form said composition, wherein said first container comprises PLGA and risperidone and said second container comprises DMSO; c) providing a pharmaceutical kit comprising at least two containers within which the ingredients of the prolonged-release injectable composition are divided and mixing the contents of a first container with the contents of a second container to form said composition, wherein said first container comprises risperidone and said second container comprises PLGA and DMSO; or d) providing a pharmaceutical kit comprising multiple containers within which the ingredients of the prolonged-release injectable composition are contained, dividing and mixing the contents of said multiple containers to form said composition, wherein said multiple containers comprise a first container comprising risperidone, another container comprising PLGA, and another container comprising DMSO. In some embodiments, the kit comprises a single dose of risperidone.

The invention provides a method of treating a disease, condition or disorder that is therapeutically responsive to risperidone and/or paliperidone, the method comprising preparing the prolonged-release injectable composition, and administering the prolonged-release injectable composition to a subject in need thereof, thereby treating said disease, disorder or condition. The method may further comprise the step of providing a pharmaceutical kit as described herein.

In some embodiments, the method of administering comprises a) providing a pharmaceutical kit comprising a container with DMSO and a container with drug and PLGA, wherein the PLGA has a particle size distribution as described herein; b) preparing a prolonged-release injectable composition as described herein by mixing the components of the containers, thereby forming the prolonged-release injectable composition; and c) administering the prolonged-release injectable composition to a subject.

In some embodiments, the method of treating comprises a) providing a pharmaceutical kit comprising a container with DMSO and a container with drug and PLGA, wherein the PLGA has a particle size distribution as described herein; b) preparing a prolonged-release injectable composition as described herein by mixing the components of the containers, thereby forming the prolonged-release injectable composition; and c) administering the prolonged-release injectable composition to a subject in need thereof.

In the methods and kits described herein, the DMSO, PLGA, and drug may be divided among two, three, or more containers in the pharmaceutical kit.

Example diseases, conditions or disorders responsive to risperidone or responsive to paliperidone include, for example, psychosis, delusional psychosis, psychotic depression, obsessive-compulsion disorder, schizophrenia, bipolar disorder, schizoaffective disorders, non-schizophrenic psychoses, Asperger's syndrome, Tourette's syndrome, obsessive-compulsion disorder, post-traumatic stress disorder, attention deficit hyperactivity disorder, personality disorders, aggression, depression, dementia, intellectual disabilities and behavioural disturbances in mental retardation and autism, autistic spectrum disorders, anxiety, eating disorders, nervous anxiety, insomnia, idiopathic dystonia, substance abuse, and any combination thereof. Treatment of episodes of acute exacerbation of the above diseases, conditions, and disorders is within the scope of the invention. Schizophrenia, schizoaffective disorder, bipolar disorder, and bipolar mania are preferred.

In some embodiments, the psychosis is an episode of acute psychosis, such as acute exacerbation of schizophrenia, wherein the risperidone-containing prolonged-release injectable composition disclosed herein is administered by intramuscular administration once about every 28 days, about every 28-32 days, or about every month.

The invention includes all combinations of the aspects, embodiments and sub-embodiments stated in this invention.

## BRIEF DESCRIPTION OF THE FIGURES

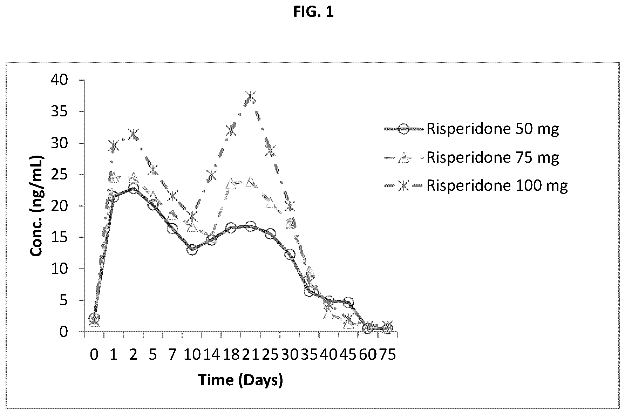

FIG. 1 represents the mean plasma concentration profile for the total active moieties (risperidone plus 9--H-risperidone) following intramuscular administration of three dosage strengths (50 mg, 75 mg, and 100 mg of risperidone) of a prolonged-release injectable composition.

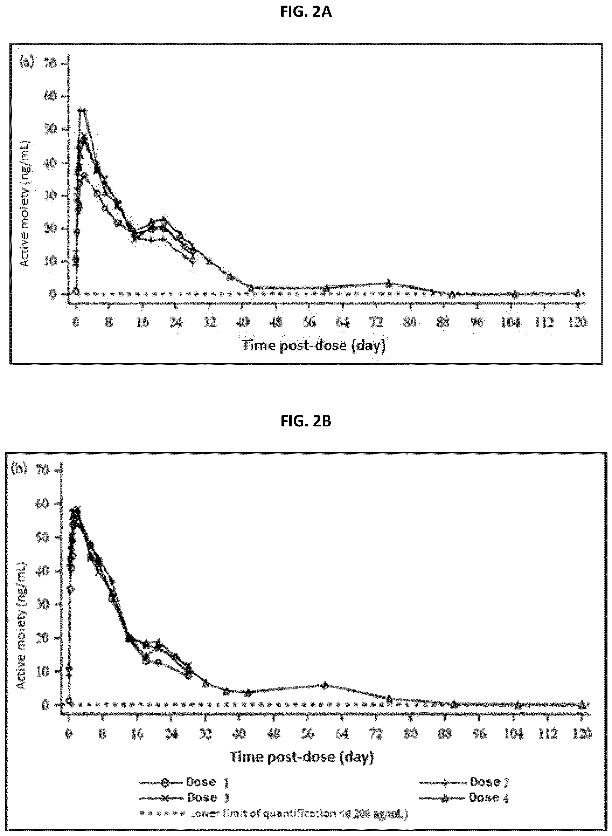

FIGS. 2A and 2B represents the mean plasma concentration profile for the total active moieties (risperidone plus 9-OH-rispeddone) following repeated (four administrations in total) once-every 28 days intramuscular administration of 75 mg of risperidone in a prolonged-release injectable composition to the gluteal muscles (FIG. 2A) and deltoid muscles (FIG. 2B).

## DETAILED DESCRIPTION OF THE INVENTION

Unless specified otherwise, the term drug, metabolite and prodrug thereof are used interchangeably. In general, the term drug encompasses a metabolite and prodrug thereof.

As used herein, the term "loading dose" or "loading doses" relates to a) oral administration of a dose of risperidone to a subject on a daily basis for a period of several days, wherein said loading doses are sufficient to establish in the subject a steady state plasma concentration of active moiety that is within a therapeutic range; and/or b) administration of one or more higher doses of prolonged-release injectable composition comprising risperidone prior to administration of a maintenance dose of prolonged-release injectable composition comprising risperidone, wherein said one or more higher doses are sufficient to establish in the subject a steady state plasma concentration of active moiety that is within a therapeutic range prior to administration of said maintenance dose.

As used herein, the term "maintenance dose" refers to an amount of prolonged-release injectable composition comprising a specified amount of risperidone, wherein said amount is administered to a subject from the first (initial) administration of said prolonged-release injectable composition through subsequent administrations (about once every 28 days or about once monthly) of said prolonged-release injectable composition. Said maintenance dose is sufficient to establish in the subject a steady state plasma concentration of active moiety that is within a therapeutic range without requiring administration of loading doses of prolonged-release injectable composition comprising risperidone and without requiring oral supplementation with risperidone.

As used herein, the term "oral supplementation" refers to oral administration of a dose of risperidone to a subject on a daily basis after the subject has been administered a prolonged-release injectable composition of the invention.

As used herein and unless otherwise specified, the drug or active ingredient included in the injectable composition can be present in free base, salt, amorphous, crystalline, anhydrous, hydrate, optically pure, optically enriched or racemic forms thereof. Combinations of these various forms are also within the scope of the invention. A prodrug, metabolite (paliperidone) or derivative of the drug can also be included.

In some embodiments, the salt forms of risperidone can be made according to U.S. publication No. 20040266791, the relevant disclosure of which is hereby incorporated for reference; however, other known salts can be used.

As used herein, the term "prodrug" is taken to mean a compound that is administered in an inactive (or less than fully active) form, and is subsequently converted to an active pharmacological agent through normal metabolic processes. A prodrug serves as a type of 'precursor' to the intended drug, e.g. risperidone, paliperidone or other drug.

As used herein, the term "derivative" is taken to mean a compound that is obtained by chemical modification of a parent compound such that the "derivative" includes therein almost all or all of the chemical structure of the parent (or base) compound. A derivative is a compound that is formed from a similar compound or a compound that can be imagined to arise from another compound, if one atom is replaced with another atom or group of atoms. A derivative is a compound derived or obtained from another and containing essential elements of the parent substance. A derivative is a chemical compound that may be produced from another compound of similar structure in one or more steps.

As used herein, the term "dosing period" refers to the period of days or weeks as measured from the initial day after administration of a dose to at least 28 days after administration or to administration of a subsequent dose. During the dosing period, the implant will provide therapeutic plasma levels of drug for about 4 weeks or more. A dosing period can end after expiration of a predetermined number of days or after the plasma level of drug drops below therapeutic levels.

As used herein, a "treatment period" refers to the weeks, months or years during which LAI depot compositions (resulting in respective implants) of the invention are administered to a subject. A treatment period generally comprises plural dosing periods. Dosing periods can occur sequentially or in an overlapping manner during a treatment period. For example, a first dose of injectable composition is administered, and a second dose of injectable composition can be administered at a time following administration of the first dose, such that each dose will have its own corresponding dosing period, and the dosing periods would overlap. Dosing periods will typically be sequential or overlap by no more than about one day up to about two, three, four, five, six or seven days.

The present invention uses a risperidone-containing composition suitable for forming one or more in situ intramuscular implants which can continuously maintain the required plasma levels of active moiety (which is risperidone and/or paliperidone) for at about 28 days or 28+/-5 days or 28+/-4 days or 28+/-3 days 28+/-2 days or 28+/-1 day or about 26-33 days or about 28-33 days. Moreover, the composition has improved pharmaceutical performance. The term "implant", as used herein, refers to compositions based on the formation of a solid and stable polymeric matrix system entrapping drug particles. This term is used to avoid confusion with the term microparticles which is commonly known as **performed polymeric microparticles obtained by solvent evaporation or spray-drying techniques.** The compositions of the invention contain drug particles, suspended in a polymer solution, that are entrapped in a solid polymer matrix formed in situ at the site of injection as the solvent diffuses and the polymer precipitates forming a matrix that contains drug particles. The term "implant" as used herein, refers to a depot composition (prolonged-release depot), since the composition of the present invention is an injectable depot composition, particularly a prolonged-release injectable composition that forms a prolonged-release depot when injected. The prolonged-release injectable composition is also considered an injectable suspension (or a prolonged-release injectable composition) comprising particles of risperidone suspended within a polymeric solution of DMSO and PLGA, wherein the prolonged-release injectable composition forms a prolonged-release depot (implant) after administration. The intramuscular dose can be administered to any muscle or muscle group typically recognised by the pharmaceutical industry as a suitable site for an injectable composition. In some embodiments, the composition is administered to the gluteal and/or deltoid muscles. The composition can also be administered to the quadriceps muscle group. A dose can be administered to a single muscle site or can be divided into two or more portions and administered to two or more muscle sites of a subject. For example, a first portion of a dose can be administered to a first section of gluteal muscle and a second portion of the dose can be administered to a second section of gluteal muscle of a subject. The injectable composition can be administered to a subject in one or more injection sites on the same day and still be considered as being part of the same dosing period. For example, part of a dose can be administered to a first injection site and another part of the same dose can be administered to another injection site. A single-body implant will form at each injection site. Such a form of administration within a same day is considered to be administration of a single dose with a single dosing period. Alternatively, administration can be modified such that there is one point of needle entry into the subject but more than one injection site below the skin, which can be achieved by making a first penetration into the skin and muscle and administering a portion of a dose, then partially withdrawing and redirecting the needle into another section of muscle, while maintaining the tip of the needle beneath the skin, and then injecting another portion of the dose into this other section of muscle. Such a mode of administration is still considered to be administration of a single dose within a single dosing period.

A therapeutically effective amount of injectable composition refers to an amount of injectable composition comprising a specified dose of drug. Accordingly, a therapeutically effective amount of 75-100 mg of injectable composition comprises a dose of 75-100 mg of risperidone; therefore, the actual amount of prolonged-release injectable composition administered would be greater than 75-100 mg, the actual amount of injectable composition being determined according to the drug content in the prolonged-release injectable composition. For example, a 75 mg dose of risperidone in a therapeutically effective amount of prolonged-release injectable composition comprising about 13% wt of risperidone would be equivalent to a therapeutically effective amount of about 577 mg, said composition comprising DMSO, risperidone, and PLGA. Similarly, a 100 mg dose of risperidone in a therapeutically effective amount of prolonged-release injectable composition comprising about 13% wt of risperidone would be equivalent to therapeutically effective amount of about 769 mg, said composition comprising DMSO, risperidone, and PLGA.

Although not required, the present injectable composition can further comprise an alkaline agent. An alkaline agent with low water solubility such as lower than 0.02 mg/ml can be included. The alkaline agent can be present in a molar ratio >2/5 (drug/alkaline agent), meaning that the alkaline agent is present in molar excess over the drug. Preferred alkaline agents are alkaline or alkaline-earth hydroxides, such as magnesium hydroxide or aluminium hydroxide. Due to the limited water solubility of the alkaline agent, the d0.5 of the particle size distribution, e.g. of the magnesium hydroxide, is preferably below 10 microns.

The method of the invention can use a pharmaceutical kit suitable for in situ formation of a biodegradable solid implant in a subject in need thereof. In some embodiments, the kit comprises: a first container comprising risperidone; a second container comprising a biocompatible PLGA copolymer; and a third container comprising DMSO. By mixing the contents of the third container with the contents of the second container, a polymeric solution is formed, which solution is then mixed with the contents of the first container to form the injectable composition as described herein. In some embodiments, the copolymer and drug (and/or a metabolite and/or a prodrug thereof) are included in a first container, and DMSO is included in a second container. In some embodiments, the drug (and/or a metabolite and/or a prodrug thereof) is included in a first container, and PLGA and DMSO are included in a second container. In some embodiments, the containers are syringes and the mixing of their contents may be performed by direct or indirect connection followed by moving the plungers of the syringes forwards and backwards. Embodiments of the invention include those wherein a) drug and/or copolymer is present in solid form in a container prior to mixing with the solvent; or b) drug and/or copolymer is present in particulate form or as a lyophilisate in a container prior to mixing with the solvent (DMSO).

In some embodiments, the prolonged-release injectable composition is included in ready-to-use form in a single container stored at room temperature or under refrigerated condition. The ready-to-use form can be provided in a single-dose or multi-dose format.

The prolonged-release injectable composition can be prepared by mixing a polymeric solution with risperidone. As used herein, the term "polymeric solution" is taken to mean the fluid composition comprising a combination of DMSO and the polymer dissolved therein. In some embodiments, at least 80%, at least 90%, at least 95%, at least 99% or all of the polymer is dissolved in the DMSO. If not specified otherwise, the viscosity value of the polymeric solution or the injectable composition is given in Pa·s units.

If not specified otherwise, the viscosity value of the polymeric solution or the injectable composition is given in Pa·s units. The polymeric solution has a viscosity in the range of around 0.5 to around 3.0 Pa·s, around 0.7 to around 3.0 Pa·s, around 0.7 to around 2.0 Pa·s, around 1.5 to around 2.5 Pa·s, around 1.5 to around 2.3 Pa·s, around 1.5 to around 2.1 Pa·s, 1.5-2.1 ±10% Pa·s, 1.6-1.9 ±10% Pa·s, or 1.7-1.8 ±10% Pa·s. Before administration, the prolonged-release injectable composition has a viscosity in the range of about 1.0-7.0 Pa·s, about 1.5-7.0 Pa·s, or about 1.8-6.5 Pa·s. In some embodiments, the values may vary about ±10% from the specified limits. The viscosity can be controlled primarily according to the molecular weight (the intrinsic or inherent viscosity) of the polymer and the concentration of polymer in the injectable composition.

In some embodiments, the mass ratio of polymeric solution to drug, expressed as the mass of (polymer + solvent) to the mass drug, ranges from about 15:1 to about 5:1, from about 12:1 to about 5:1, from about 7:1 to about 6.5:1, from about 6.5:1 to about 6.8:1, about 6.67:1, about 6.66:1, or about 6.68:1. In some embodiments, the mass ratio of polymer to polymeric solution, expressed as the weight percentage of polymer with respect to the weight of polymer + solvent, is about 25-50%, about 25-35%, about 30-40%, about 28-32%, or about 30%.

In some embodiments, the mass ratio of solvent (DMSO) to polymeric solution, expressed as the weight percentage of solvent with respect to the weight of polymer + solvent, is about 50-75%, about 65-75%, about 60-70%, about 68-72%, or about 70%.

The LAI depot compositions used in the method of the invention may comprise at least one polymer (or copolymer), DMSO, and risperidone. They may further comprise one or more pharmaceutical excipients suitable for intramuscular administration.

Following intramuscular administration, the prolonged-release injectable composition forms an in situ solid implant in the muscle tissue. When the implantable compositions are exposed to body fluids or water, the solvent (DMSO) diffuses far away from the polymer-drug mixture and the polymer precipitates thereby trapping or encapsulating the drug within the polymeric matrix as the composition solidifies into a single implant at the injection site. The release of drug follows the general characteristics for diffusion or dissolution of a drug from within a polymeric matrix. The drug is also released by polymer erosion/degradation. The drug (active ingredient) forms a suspension or dispersion within a biodegradable and biocompatible polymeric solution to form an injectable composition that can be administered by way of a syringe (or pump) and a needle. In some embodiments, the implant starts the release of risperidone within about 2 hours after administration to provide a rapid (e.g. less than 1 day, less than 18 hours, less than 12 hours, less than 6 hours, less than 3 hours) onset of action and continuously for at least 4 weeks. It provides therapeutically effective plasma levels of active moiety (risperidone + 9-OH-risperidone) from the first day of administration for a period of at least 4 weeks.

The expression "about 50:50" as used in this description, refers to a monomer ratio of lactic to glycolic acid of biocompatible PLGA copolymer based on lactic and glycolic acid which is applied in the context of the invention for a monomer ratio measure with a standard technical error of ± 10%. The commercially available grades of PLGA copolymer are known to vary slightly in their actual ratio of monomers even though they may be listed as having a 50:50 monomer ratio. For example, a copolymer specified as having a monomer ratio of 50:50 may actually have a monomer ratio ranging from 45:55 to 55:45 or 48:52 to 52:48. Accordingly, whenever the monomer ratio of "50:50" or "about 50:50" is specified herein, all ratios ranging from 45:55 to 55:45 are considered as being interchangeable therewith.

The compositions of the invention comprise a biodegradable poly(L-lactide-co-glycolide) copolymer (PLGA). The monomer ratio (L:G) of lactic acid to glycolic acid monomers present in the polymer can range from about 35:65 to about 75:25, from about 50:50 to about 75:25, from about 45:55 to about 70:30, from about 50:50 to about 65:35, from about 65:35 to about 75:25, or said ratio can be 50:50 ±10% or 75:25 ±10%.

Inherent viscosity can be measured in chloroform at 25°C or 30°C at a concentration of 0.1% w/v or 0.5% with a size 0c or 0B Ubbelohde glass capillary viscometer (RESOMER® grades) or in chloroform at 30°C and at a concentration of 0.5% w/v with a size 25 Cannon-Fenske glass capillary viscometer. Suitable grades of PLGA copolymers as described herein (according to molecular weight, intrinsic viscosity and/or molar ratio of lactic acid monomer to glycolic acid monomer) are end-capped (such as with an ester group, e.g. lauryl ester, methyl ester) are available FROM EVONIK® (Essen, Germany), Boehringer Ingelheim (Ingelheim am Rhein, Germany), ALKERMES (Dublin, Ireland) or SIGMA ALDRICH (St. Louis, MO) and are marketed under the trade names RESOMER®, LAKESHORE BIOMATERIALS™ or MEDISORB®. As the composition of some grades of end-capped PLGA is proprietary, the identity of the ester end-cap is not publicly available. Nonetheless, the performance properties of the grades of PLGA copolymer described herein are known and are used to characterise the material.

For the purpose of the present invention, throughout the present specification the term inherent viscosity ($\eta_{inh}$) of the polymer is defined as the ratio of the natural logarithm of the relative viscosity, $\eta_r$, with respect to the mass concentration of the polymer, c, i.e.:

$$\eta_{inh} = (\ln\eta_r)/c$$

and the relative viscosity ($\eta_r$) is the ratio of the viscosity of the solution $\eta$ with respect to the viscosity of the solvent $\eta_s$, i.e.:

$$\eta_r = \eta / \eta_s$$

If not otherwise specified, the inherent viscosity and molecular weight values throughout the present specification are to be understood as measured with the method explained in example 1 (Method A and/or Method B). The value of inherent viscosity is considered in the present specification, as commonly accepted in the art, as an indirect indicator of the polymer molecular weight. In this way, a reduction in the inherent viscosity of a polymer, measured at a given concentration in a certain solvent, with same monomer composition and terminal end groups, is an indication of a reduction in the polymer molecular weight (IUPAC. Basic definitions of terms relating to polymers 1974. Pure Appl. Chem. 40, 477-491 (1974)).

The PLGA polymer in the prolonged-release injectable composition can have an inherent viscosity in the range of 0.20-0.60 dl/g, about 0.30-0.55 dl/g, about 0.36-0.52 dl/g, about 0.40-0.58 dl/g, or about 0.46-0.51 dl/g measured in chloroform at 30°C and at a concentration of 0.5% wt with a size 0B Ubbelohde glass capillary viscometer.

The PLGA polymer in the prolonged-release injectable composition can have an average or mean molecular weight ranging from about 27-47 kDa, about 31-43 kDa, about 31-40 kDa, about 30-46 kDa, or about 30-36 kDa.

The PLGA polymer can be irradiated with a beta or gamma radiation at a dose of about 10 to about 30 kGy at a temperature between -40°C and +35°C. Irradiation can serve to reduce the molecular weight of and/or to sterilise the PLGA polymer. In some embodiments, the polymer is irradiated at a temperature lower than 35°C, more preferably lower than 25°C and more preferably lower than 8°. In a preferred embodiment of the invention, the biocompatible copolymer is gamma or beta irradiated in the dose range of 10-30 kGy ±10% measured at a temperature between -40°C to +35°C to adjust its molecular weight to range from about 27-47 kDa, about 31-43 kDa, about 31-40 kDa, about 30-46 kDA, or about 30-36 kDa. In a more preferred embodiment, the polymer is irradiated at 15-25 kGy ±10% measured at the temperature of 8°C.

The concentration of the polymeric component in the compositions of the invention can be in the range of 20-50%, 24-50%, 24-34%, about 24-30%, about 25-27% or about 26% (expressed as the percentage of polymer weight based on total formulation weight). In some embodiments, at least 80%, at least 90%, at least 95%, at least 99% or all of the polymer is dissolved in the DMSO or injectable composition.

In some embodiments, the drug content ranges from about 4% to about 16% wt, about 7% to about 15% wt, about 10% to about 15% wt, about 12% to about 14% wt, or about 13% wt.

After administration, the injectable composition forms an implant that provides a satisfactorily controlled release profile for the drug. By "satisfactorily controlled" release profile it is understood that that the implant will exhibit an initial release profile that is not too steep (fast), which would otherwise lead to plasma levels that are too high with concomitant toxic side effects, and an initial release profile that is not too flat (slow), which would lead to plasma levels that are below therapeutic concentrations. An implant having a satisfactorily controlled initial release profile will release no more than 20% wt, no more than 15% wt, no more than 12% wt, no more than 10% wt, no more than 8% wt, no more than 6% wt, no more than 5% wt, no more than 4% wt, no more than 3% wt, no more than 2% wt or no more than 1% wt of its charge of drug within 24 hours after being placed in an aqueous environment. It will release at least 0.1% wt, at least 0.5% wt, at least 1% wt, at least 2% wt., at least 3% wt or at least 4% wt of its charge of drug within 24 hours after being placed in an aqueous environment. The invention includes all combinations of the embodiments herein.

The plasma concentration profile during the dosing period can exhibit one, two, or more maxima and one, two or more minima. An initial maximum can be caused by dissolution of drug during the initial day(s) of the dosing period followed by a slowing of the release thereof and another maximum can be caused by increased rate of release during the remaining days of the dosing period. Embodiments of the invention include those wherein: a) the plasma profile exhibits a maximum during the initial six days or initial three days or initial two days or initial one to two days of the dosing period; b) the plasma profile exhibits a maximum during the latter 10 to 24 days of a 4-week dosing period; c) the plasma profile exhibits a maximum during the initial days of the dosing period and a maximum during the remaining days of the dosing period; d) the plasma profile is substantially level (a standard deviation within ±30%, ±25%, ±20%, ±15%, ±10% or ±5% of the average or mean) during the dosing period; e) the plasma profile exhibits a maximum during the initial 48 hours or initial 72 hours or initial 24 to 48 hours of the dosing period; and/or f) the plasma profile exhibits a maximum during the latter 10 to 28 days or latter 18 to 25 days of a 4- to 5-week dosing period.

In humans, the average plasma concentration of active moiety (risperidone + 9-OH-risperidone) can range from about 3-200, from about 5-80, or from about 10-60 ng/ml when an amount of injectable composition equivalent to a dose of about 20-80 mg, about 37.5-125 mg, or about 50-100 mg of risperidone is administered. The mean Cmin during the dosing period is in the range of about 1-80, 5-50, or about 5-40 ng/ml when an amount of injectable composition equivalent to a dose of about 25-150, about 37.5-125, or about 50-100 mg, respectively, of risperidone is administered. The average Cmax during the dosing period is in the range of about 8-300, 10-150, or 10-120 ng/ml when an amount of injectable composition equivalent to a dose of 25-150, 37.5-125, or 50-100 mg, respectively, of risperidone is administered. Some individual subjects may, on an equivalent dose basis, exhibit plasma concentrations outside the ranges specified herein for reasons such as poor health, advanced age, compromised metabolism, renal failure, disease, etc. Even so, a majority of subjects in a subject population to which the injectable implant is administered will show plasma concentrations with those specified herein.

As used herein, whenever the plasma concentration of a drug is mentioned, such plasma concentration includes within it the sum total of the plasma concentration of the drug and its active metabolite(s). For example, whenever the plasma concentration of risperidone is mentioned, such plasma concentration includes within it the sum total of the plasma concentrations of risperidone and its active metabolite(s), such as 9-OH-risperidone (paliperidone).

In some embodiments, the particle size distribution of the drug is as follows: not more than 10% of the total volume of drug particles are less than 10 microns in size (equivalent diameter in volume as a function of applying Fraunhofer theory to irregularly shape particles; as measured by laser light scattering, such as with a Malvern Mastersizer 2000) and not more than 10% of the total volume of drug particles are greater than 225 microns (or 235 microns) in size. In addition, the drug particles have a value of d0.5, preferably in the range of about 60-130 microns. Accordingly, in some embodiments, the risperidone comprises a broad particle size distribution, which can be monomodal, bimodal or trimodal. In some embodiments, the drug exhibits one of the following particle size distributions:

| Parameter | I | II | III | IV | V | VI |
|---|---|---|---|---|---|---|
| d0.1 (microns) | 27.49 | <30 | 17.41 | ≤20 | ≤10 | ≤10 |
| d0.5 (microns) | 79.90 | 40-130 | 51.61 | 40-130 | 40-130 | 40-130 |
| d0.9 (microns) | 176.66 | > 170 | 175.32 | > 170 | >225 or >235 | > 200 |

In a preferred embodiment of the invention, this drug has the particle size distribution as follows:

- not more than 10% of the total volume of particles is less than 10 microns in size;
- not more than 10% of the total volume of particles is greater than 225 microns (or 235 microns) in size or not more than 10% of the total volume of particles is greater than 200 microns in size, and
- the d0.5 of the size distribution is in the range of about 60-130 microns, about 40-90 microns, or about 40-130 microns.

The particle size distribution was determined by light scattering technique using laser light diffraction in wet mode.

Embodiments of the invention include those wherein: a) the risperidone is present in solid form in the container prior to mixing with the solvent; b) the risperidone is present in particulate form or as a lyophilisate in the container prior to mixing with the solvent; c) the particle size distribution of the risperidone is as follows: not more than 10% of the total volume of drug particles are less than 10 microns in size and not more than 10% of the total volume of drug particles are greater than 225 microns (or 235 microns) in size; d) the d0.5 of the particle size distribution is in the range of about 60-130 microns; e) the mass ratio of the amount of polymeric solution (polymer + solvent) and the amount of risperidone in the injectable composition ranges from about 15:1 to 5:1; f) the mass ratio of the amount of solvent and the amount of risperidone (mg of solvent/mg of risperidone) in the injectable composition ranges from about 12:1 to 4:1; g) the kit further comprises an alkaline agent; h) the mole ratio of risperidone to alkaline agent ranges from 2/3 to 2/5; i) the solvent, polymeric solution, risperidone and/or injectable composition is sterilised prior to administration; and/or j) the kit further comprises an alkaline agent in either or both containers.

Additional parameters such as the mass ratio of drug to polymeric solution (polymer + solvent), the mass ratio of drug to (polymer + drug), the mass ratio of solvent/drug, the mass ratio of polymer to polymeric solution (polymer + solvent), the mass ratio of solvent to polymeric solution (polymer + solvent), can also be useful to provide control over the initial release and/or controlled release of drug from the compositions of the invention.

In some embodiments, the drug is partially suspended in the composition and has a solubility in DMSO below about 10 mg/ml. In some embodiments, the drug is partially dissolved or substantially completely undissolved in the solvent, DMSO, polymeric solution or injectable composition. In some embodiments, ≤2.5%, <5%, ≤7.5%, ≤10%, ≤20% or <25%, of the drug is dissolved in the solvent or polymeric solution to form the injectable composition. In some embodiments, >0%, ≥0.5%, ≥1%, ≥5%, ≥ 10% or ≥ 15% or up to about 20% wt. of the drug is dissolved in the solvent or polymeric solution to form the injectable composition. All combinations of these embodiments are contemplated.

In yet another embodiment, the composition is a sterile composition. The composition may be sterilised by sterile filtration of the polymeric solution through a filtration medium having a nominal pore size of 0.22 microns or less, or by irradiation, or by a combination thereof.

The prolonged-release injectable composition can also be used to treat episodes of acute psychosis selected from the group consisting of delusional psychosis, psychotic depression, obsessive-compulsion disorder, schizophrenia, bipolar disorder, schizoaffective disorders, non-schizophrenic psychosis, Asperger's syndrome, Tourette's syndrome, obsessive-compulsion disorder, post-traumatic stress disorder, attention deficit hyperactivity disorder, personality disorders, aggression, depression, dementia, intellectual disabilities and behavioural disturbances in mental retardation and autism, autistic spectrum disorders, anxiety, eating disorders, nervous anxiety, insomnia, idiopathic dystonia, substance abuse, and any combination thereof. The injectable composition can also be used as an antihistaminic for the treatment of allergic disorders or as a prolactin secretion promoter for breastfeeding women or for the treatment of prolactin deficiency.

According to another aspect, the invention provides a pharmaceutical kit suitable for the in situ formation of a biodegradable implant in a body from the composition claimed, wherein the drug and the biocompatible polymer are contained in a first container, and the solvent is contained in a second, separate container. Preferably, at least one of the first and second containers is a syringe, a vial, a device or a cartridge, either disposable or not and more preferably both the first and the second containers are disposable syringes. This aspect of the invention relates to a kit comprising a first container, preferably syringes, vials, devices or cartridges, all of them either being disposable or not, containing a polymer in solid form, such as PLGA and a drug in the appropriate amounts and a second container, likewise preferably syringes, vials, devices or cartridges, all of them being either disposable or not, containing the water-miscible solvent. When required, the contents of both containers are combined, for example by means of a connector or by means of male-female syringes, and mixed together so that the compositions according to the invention are reconstituted, for example by moving the plungers of the syringes forwards and backwards. Illustrative preferred embodiments include syringes connected through a connector device and syringes connected through a direct thread.

According to another aspect, the invention provides a dosing regimen method for administering an injectable intramuscular depot composition according to the invention to a patient in an episode of acute psychosis, the method comprising:

a) administering intramuscularly to the patient experiencing an episode of acute psychosis a first dose in the amount of 75 mg to 100 mg of risperidone in the prolonged-release injectable composition;
b) administering intramuscularly to said patient 75 mg to 100 mg of risperidone in the prolonged-release injectable composition, at a point of time on the 28th day, or on the 26th day to the 31st day counting from the previous administration day; and
c) repeating step b) whenever required.

Within a treatment period, administered doses of injectable composition are typically about the same.

According to an embodiment, the drug /(polymer+drug) mass ratio is about 33%, the drug content is about 13% w/w of the total formulation, and the solution viscosity of the solution between polymer and DMSO is in the range of 1.5-2.5 Pa·s, more preferably in the range of 1.5-2.1 Pa·s and even more preferably in the range of 1.7 - 1.8 P.a.s.

According to another aspect, the invention provides a dosing regimen method for intramuscularly administering a prolonged-release injectable composition according to the invention to a patient experiencing a first-time or recurrent episode of acute exacerbation of schizophrenia, the method comprising

a) administering intramuscularly to the patient experiencing an episode of acute psychosis a first dose in the amount of 75 mg to 100 mg of risperidone in the prolonged-release injectable composition;
b) administering intramuscularly to said patient 75 mg to 100 mg of risperidone in the prolonged-release injectable composition, at a point of time on the 28th day, or on the 26th day to the 31st day counting from the previous administration day; and
c) repeating step b) whenever required.

Administration of a single dose is typically considered that amount of injectable composition administered to a subject within a period of up to 24 hours, up to 12 hours, up to 6 hours, up to 3 hours, up to one hour, up to 30 minutes, up to 15 minutes or up to 5 minutes.

A dose can be administered to a single muscle site or can be divided into two or more portions and administered to two or more muscle sites of a subject. For example, a first portion of a dose can be administered to a first section of gluteal muscle and a second portion of the dose can be administered to a second section of gluteal muscle of a subject.

As used herein the term, "initial burst" or "initial release" refers to the addition of the plasma levels of drug plus those of active metabolite, which addition is also called "the active moiety" (risperidone and paliperidone together) throughout the present specification, from the moment of injection/administration of the injectable composition to a subject in need thereof until completion of the third day after the administration. For example, the drug can be risperidone and its metabolite can be paliperidone. In some embodiments, the initial period of release is within three days, within two days, within one day, within 12 hours, within 6 hours or within 2 hours after administration.

A subject being administered or being treated with a CYP2D6 enzyme inhibitor is administered a limited dose of risperidone-containing prolonged-release injectable composition as defined herein. Example CYP2D6 enzyme inhibitors are divided into three categories: strong inhibitor such as bupropion, fluoxetine, paroxetine, quinidine and terbinafine; moderate inhibitor such as abiraterone, cinacalcet, duloxetine, lorcaserin, and mirabegron; and weak inhibitor such as amiodarone, celecoxib, cimetidine, clobazam, cobicistat, escitalopram, fluvoxamine, labetalol, ritonavir, sertraline, and vemurafenib. Acceptable doses and dosing regimens for such compounds are well known and detailed in respective regulatory package leaflets. Said doses and dosing regimens are disclosed in the websites of the U.S.F.D.A. (www.fda.gov) and E.M.A. (ema.europa.eu) and other such regulatory agencies.

Unlike daily administration of oral risperidone, the method of the invention allows treatment of subjects with a fixed dose of risperidone-containing prolonged-release injectable composition independent of the dose of CYP2D6 enzyme inhibitor administered. In one embodiment, a subject in need thereof is administered by intramuscular injection a dose of about 75 mg of risperidone in a prolonged-release injectable composition as defined herein. Within about 2-4 weeks of said injection, treatment of the subject with a CYP2D6 commences. During a treatment period comprising plural dosing periods, the subject is administered doses of about 75 mg of said prolonged-release injectable composition at about 4-week intervals. The dose of CYP2D6 is adjusted as required to provide its target therapeutic benefit; however, the dose of risperidone is kept substantially consistent at about 75 mg throughout the treatment period.

For example, example approved dosing regimens for fluoxetine (PROZAC) include the following.

| Indication | Adult | Paediatric |
| --- | --- | --- |
| Major depressive disorder | 20 mg/day in am (initial dose) | 10 to 20 mg/day (initial dose) |
| Obsessive compulsive disorder | 20 mg/day in am (initial dose) | 10 mg/day (initial dose) |
| Bulimia nervosa | 60 mg/day in am | - |
| Panic disorder | 10 mg/day (initial dose) | - |
| Depressive episodes associated with bipolar I disorder | Oral in combination with olanzapine: 5 mg of oral olanzapine and 20 mg of fluoxetine once daily (initial dose) | - |
| Treatment-resistant depression | Oral in combination with olanzapine: 5 mg of oral olanzapine and 20 mg of fluoxetine once daily (initial dose) | - |

The above dosing regimens can be used in combination with the dose of about 75 mg of risperidone in the prolonged-release injectable composition.

Alternatively, a subject that is undergoing treatment with a CYP2D6 enzyme inhibitor and that has been stabilised with up to 8 mg of risperidone per day is switched to treatment with the prolonged-release injectable composition by discontinuing oral administration of risperidone and within 24 hours administering to said subject by intramuscular injection a dose of about 75 mg of risperidone in a prolonged-release injectable composition as defined herein. During a treatment period comprising plural dosing periods, the subject is administered doses of about 75 mg of said prolonged-release injectable composition at about 4-week intervals. The dose of CYP2D6 may be adjusted as needed to provide its target therapeutic benefit; however, the dose of risperidone is kept substantially consistent at about 75 mg throughout the treatment period.

By further way of example, approved drug regimens for paroxetine (PAXIL) immediate release or rapid release dosage form include the following.

| Indication | Adult |
| --- | --- |
| Major depressive disorder | 20 mg/day (initial dose); 20-50 mg/day |
| Obsessive compulsive disorder | 20 mg/day in am (initial dose); target 40 mg daily; 20-60 mg/day |
| Panic disorder | 20 mg/day (initial dose); target 40 mg/day; 10-60 mg/day |

(continued)

| Indication | Adult |
|---|---|
| Social anxiety disorder | 20 mg/day (initial dose); 20-60 mg/day |
| Generalised anxiety disorder | 20 mg/day (initial dose); 20-50 mg/day |
| Post-traumatic stress disorder | 20 mg/day (initial dose); 20-50 mg/day |

By way of further example, approved drug regimens for paroxetine (PAXIL) controlled release dosage form include the following.

| Indication | Adult |
|---|---|
| Major depressive disorder | 25 mg/day (initial dose); 25-62.5 mg/day |
| Panic disorder | 12.5 mg/day (initial dose); 12.5-75 mg/day |
| Social anxiety disorder | 12.5 mg/day (initial dose); 12.5-37.5 mg/day |
| Premenstrual dysphoric disorder | 12.5 mg/day (initial dose); 12.5-25 mg/day |

The SSRI antidepressant may be administered as an oral tablet or oral suspension. Suitable example dosage forms are described in the package leaflets for PAXIL and PROZAC.

subjects were treated according to Example 10 with the prolonged-release injectable composition of Example 11. Concomitant medications were taken by 56.5% of all patients during the study. The most common pharmacological groups were anxiolytics, anti-inflammatory and antirheumatic products, non-steroidal anti-inflammatory agents, and other analgesics and antipyretics. The data obtained in the study demonstrate the efficacy, safety, and tolerability of the risperidone-containing prolonged-release injectable composition and its method of use according to the invention in the monthly treatment of acute schizophrenia. Superiority of active treatment versus placebo was shown for the primary efficacy outcome.

Doses of risperidone were well tolerated. The adverse events (AEs) observed were those expected for oral and LAI risperidone at therapeutic doses and were consistent with that observed in previous studies. All Treatment Emergent Adverse Events (TEAEs) were mainly mild or moderate in most patients in both treatment groups. Although the frequency of TEAEs was lower with placebo than in the risperidone groups, the rate was similar to those reported in a similar study in acute schizophrenia, and slightly lower than those observed in previous LAI risperidone study. Furthermore, both risperidone groups were associated with lower rate of discontinuation owing to TEAE compared with placebo, and no patient died owing to a TEAE during the study.

Generally, the incidence of serious TEAEs and of TEAEs, leading to study drug discontinuation was low and no clear differences between treatment groups were observed. Similarly, the frequency of ISRs (redness, swelling, or induration) was low overall, with redness being the most frequent in all treatment groups, and with a slight tendency towards a dose-dependent increase of ISRs. No relevant differences between treatment groups were seen in the 0-10 Visual Analog Scale (VAS) score, with a mean value of 2.0 in all treatment groups, which is a clinically significant result for a new LAI formulation. Similarly, the EPS, akathisia, dyskinesia, and suicidality safety scales also did not indicate significant differences between either dose of risperidone and placebo. Furthermore, there were no significant differences in laboratory measurements between treatment arms from baseline through to the end of the study and no notable changes in either treatment arm, except for prolactin. The events related to the prolactin increase were among the most frequently reported TEAEs in this study, with a comparable incidence to that described by others. Several limitations need to be considered when interpreting the study results. The term "around/about" is intended to mean $\pm20\%$, $\pm 15\%$, $\pm 10\%$, $\pm 5\%$, $\pm 2.5\%$ or $\pm1\%$ relative to a specified value, i.e. "around/about 20%" means $20\pm4\%$, $20\pm3\%$, $20\pm2\%$, $20\pm1\%$, $20\pm0.5\%$, or $20\pm0.2\%$.

All values disclosed herein may have standard technical measurement error (standard deviation) of $\pm 10\%$.

**EXAMPLES**

In view of the above description and the examples and claims below, a skilled person will be able to put the invention into practice as claimed without undue experimentation. The foregoing will be better understood with reference to the following examples that detail certain methods for the preparation of embodiments of the present invention. All references made to these examples are for the purposes of illustration. The following examples should not be considered exhaustive, but merely illustrative of only a few of the many embodiments contemplated by the present invention.

Also, without limitation and in connection with the examples, acceptable plasma levels of active moiety during the initial burst phase are below 75 ng/ml in Beagle dogs when the doses administered are 2.5 mg active moiety/kg body weight.

**Example 1: Measurement of inherent viscosity**

Method A:

Equipment

GPC chromatograph with triple detector (laser diffraction, viscometry, refraction index)

- Viscotek® GPCmax VE 2001 GPC SOLVENT/SAMPLE MODULE

- Viscotek® TDA 305 TRIPLE DETECTOR ARRAY

Reagents

- • Tetrahydrofuran (THF) GPC grade stabilised with butyl hydroxyl toluene (BHT) 250 ppm
- Polystyrene narrow standard (preferable about a molecular weight of 90 or 99 KDa)

Sample preparation

- 1-2 mg/ml Standard sample
- 10 mg/ml Test sample 3 samples for each polymer to be tested.

Pre-conditioning

Condition and stabilise column and detectors with mobile phase (THF) until reaching working flow rate of 1 ml/min and purge viscometer and refraction index detectors, checking at the end that all signals are stable and suitable.

Chromatographic conditions:

- Column 2 serial columns i-MBMMW-3078 (CLM1012, Viscotek)
- Delay column: medium delay (CLM9002, Viscotek)
- Column temperature 30°C
- Flux rate 1 ml/min
- Injection volume 100 $\mu$l
- Run time: 35 minutes
- Eluent: Stabilised THF (pre-heated to 30°C and under 100 rpm agitation)

System verification

- Inject 100 $\mu$l of eluent and verify that there is no response in signals related to molecular weight determination
- Inject 100 $\mu$l of polystyrene narrow standard and verify suitability of the measurement. Repeat at least twice.

Acceptance criteria: $\pm$ 5% of the nominal molecular weight and $\pm$3% intrinsic viscosity declared by the manufacturer's standard certificate.

Calibration

Not necessary if system verification complies and no previous chromatographic conditions are changed. If necessary to calibrate:

- Inject 100 $\mu$l of polystyrene standard at least twice.
- Use first sample's data for triple calibration by creating a new multidetector - homopolymer method.
- Enter into the method all the data needed for internal calibration such standard values of MW, IV, dn/dc, dA/dc and refractive index of the solvent.
- Calibrate the system as the equipment specifies and save the new method.
- Check with the new method the suitability of the measurement for the second injection of the standard.

Method

Inject (in triplicate) 100 $\mu$l of the test sample

The polymer's molecular weight measured according to the technique specified resulted in 32.5 KDa. According to a similar technique, inherent viscosity of the polymer resulted in a value of 0.27 dl/g. It is important to mention that inherent viscosity values correspond to those obtained with the technique described, especially related to temperature conditions and eluent used. Any change in measurement conditions mean different values are obtained as they directly depend on them.

Method B:

Equipment

Automated capillary viscometry

- Rheotek® -RPV-2 automatic polymer viscometer

Reagents

- Chloroform (HPLC grade)
- Acetone

Sample preparation

- 0.5 mg/ml Test sample filtered: 2 samples for each polymer to be tested.

Conditions

The test is conducted at the temperature of $30.0 \pm 0.1°C$

Method

Measure the solvent in the first time and then the sample. Filtered chloroform (solvent) or sample: Using a glass syringe, filter the solvent or sample through a 0.45 $\mu$m PTFE filter discarding the first ml.
Inject 12 to 15mL of solvent or sample. Do not overfill the viscometer tube. The solvent or sample should be between lines as shown. It is important to mention that inherent viscosity values correspond to those obtained with the technique described, especially related to the conditions of temperature, concentration and solvent used. Any change in measurement conditions mean different values are obtained as they directly depend on them.

**Example 2: Depot formulation with Resomer® 503 without irradiation**

In the present example, the following formulation was prepared:

| | Ingredient | Amount (mg) |
|---|---|---|
| Female 2.25 ml syringe | Lactic-co-glycolic acid copolymer (N-capped) with 50% content of each of the two organic acid monomers and a molecular weight of 32 KDa. | 50 |
| | Risperidone | 25 |
| Male 2.25 ml syringe | Ingredient | Amount (mg) |
| | Dimethylsulfoxide | 117 |

Risperidone particle size was characterised by light scattering and provided the following distribution of particle size: d(0.1) = 27.49 μm, d(0.5) = 79.90 μm and d(0.9) = 176.66 μm.

**Example 3: Depot formulation with Resomer® 504 irradiated at 16 KGy.**

The present example shows how the polymer molecular weight can be controlled in order to have a sterile formulation with the desired *in vivo* release properties.

Filling solid polymer in syringes represents a real challenge in the manufacturing of injectable formulations. The polymer, manufactured as a non-sterile product, requires sterilisation to achieve a formulation that can be injected into human beings. Probably the best way to resolve this technical issue is to subject the polymer to sterilisation by gamma or beta irradiation. Irradiation represents a challenging problem when biodegradable polymers are used, as irradiation can disrupt the chains into fractions of smaller size. Control of the polymer molecular weight again appears as the critical parameter to control the final characteristics of a product after a sterilisation process.

However, chain size reduction by irradiation can be mathematically modelled or controlled in order to predict the final molecular weight of a polymer to be used as raw material having a molecular weight higher than desired. Therefore, after determining the fill weight of the polymer to be filled in a container (for example, the fill weight of the polymer in a syringe) and the bioburden present in the polymer as raw material, the irradiation dose required to get the polymer sterile (as specified by ISO 11137) is selected for the required fill weight.

Then the mathematical model describing the loss of molecular weight for a certain polymer versus the irradiated dose can identify the initial molecular weight of the polymer to be used as raw material required to obtain, after the irradiation process, a polymer with the desired final molecular weight for the formulation.

As the availability of a polymer with a specific molecular weight can be somewhat limited, it is thus possible to select an available polymer with a molecular weight that is higher than that required according to the irradiation dose identified, and then adjust the irradiation dose to a higher value in order to obtain a sterile polymer with the required molecular weight. In this example, a lactic-co-glycolic acid copolymer with 50% content of each of the two organic acid monomers and a molecular weight of 38 KDa was sterilised by beta irradiation at 16 KGy under controlled temperature and humidity conditions. The resultant polymer was characterised by its molecular weight according to the method described in example 1. The molecular weight after the irradiation process was 31 KDa.

| | Ingredient | Amount (mg) |
|---|---|---|
| Female 2.25 ml syringe | Lactic-co-glycolic acid copolymer (N-capped) with 50% content of each of the two organic acid monomers and a molecular weight of 38 KDa, beta-irradiated in bulk with a 16 KGy dose achieving a final molecular weight of 31 KDa. | 50 |
| | Risperidone | 25 |
| Male 2.25 ml syringe | Ingredient | Amount (mg) |
| | Dimethylsulfoxide | 117 |

Risperidone particle size was characterised by light scattering and provided the following distribution of particle size: d(0.1) = 27.49 μm, d(0.5) = 79.90 μm and d(0.9) = 176.66 μm.

Inherent viscosity of the irradiated polymer, as calculated by the technique described in example 1 was 0.27 dl/g.

The risperidone implantable formulation was prepared by connecting male and female syringes and moving the plungers forwards and backwards upon complete dissolution of the polymer and the formation of a homogeneous suspension of the risperidone in the polymer solution.

**Example 4: Depot formulation with Resomer® 504 irradiated at 25 KGy.**

This is another example that shows how the polymer's molecular weight can be controlled in order to have a sterile formulation with the desired *in vivo* release properties.

A lactic-co-glycolic acid copolymer with 50% content of each of the two organic acid monomers and a molecular weight of 50 KDa was sterilised by beta irradiation at 25 KGy under controlled temperature and humidity conditions. The resultant polymer was characterised by its molecular weight according to the method described in example 1. The molecular weight after the irradiation process was 35 KDa.

| | Ingredient | Amount (mg) |
|---|---|---|
| Female 2.25 ml syringe | Lactic-co-glycolic acid copolymer (N-capped) with 50% content of each of the two organic acid monomers and a molecular weight of 50 KDa, beta-irradiated in bulk with a 25 KGy dose achieving a final molecular weight of 35 KDa. | 50 |
| | Risperidone | 25 |
| Male 2.25 ml syringe | Ingredient | Amount (mg) |
| | Dimethylsulfoxide | 117 |

Risperidone particle size was characterised by light scattering and provided the following distribution of particle size: d(0.1) = 27.49 $\mu$m, d(0.5) = 79.90 $\mu$m and d(0.9) = 176.66 $\mu$m.

Inherent viscosity of the irradiated polymer, as calculated by the technique described in example 1 was 0.28 dl/g.

The risperidone implantable formulation was prepared by connecting male and female syringes and moving the plungers forwards and backwards upon complete dissolution of the polymer and the formation of a homogeneous suspension of the risperidone in the polymer solution.

**Example 5: Depot formulation with Lakeshore Biomaterials® 5050 DLG 5E irradiated at 25 KGy**

This is another example that shows how the polymer's molecular weight can be controlled in order to have a sterile formulation with the desired *in vivo* release properties.

A lactic-co-glycolic acid copolymer with 50% content of each of the two organic acid monomers and a molecular weight of 56 KDa was sterilised by beta irradiation at 25 KGy under controlled temperature and humidity conditions. The resultant polymer was characterised by its molecular weight according to the method described in example 1. The molecular weight after the irradiation process was 45 KDa.

| | Ingredient | Amount (mg) |
|---|---|---|
| Female 2.25 ml syringe | Lactic-co-glycolic acid copolymer (N-capped) with 50% content of each of the two organic acid monomers and a molecular weight of 56 KDa, beta-irradiated in bulk with a 25 KGy dose achieving a final molecular weight of 45 KDa. | 50 |
| | Risperidone | 25 |
| Male 2.25 ml syringe | Ingredient | Amount (mg) |
| | Dimethylsulfoxide | 117 |

Risperidone particle size was characterised by light scattering and provided the following distribution of particle size: d(0.1) = 27.49 $\mu$m, d(0.5) = 79.90 $\mu$m and d(0.9) = 176.66 $\mu$m.

Inherent viscosity of the irradiated polymer, as calculated by the technique described in example 1 was 0.28 dl/g.

The risperidone implantable formulation was prepared by connecting male and female syringes and moving the plungers forwards and backwards upon complete dissolution of the polymer and the formation of a homogeneous suspension of the risperidone in the polymer solution.

**Example 6: Depot formulation with Resomer® 504 irradiated at 25 KGy.**

In this example, a lactic-co-glycolic acid copolymer with 50% content of each of the two organic acid monomers and a molecular weight of 38 KDa was sterilised by beta irradiation at 25 KGy under controlled temperature and humidity conditions. The resultant polymer was characterised by its molecular weight according to the method described in example 1. The molecular weight after the irradiation process was 28 KDa.

| Female 2.25 ml syringe | Ingredient | Amount (mg) |
|---|---|---|
| | Lactic-co-glycolic acid copolymer (N-capped) with 50% content of each of the two organic acid monomers and a molecular weight of 38 KDa, beta-irradiated in bulk with a 25 KGy dose achieving a final molecular weight of 28 KDa. | 50 |
| | Risperidone | 25 |
| Male 2.25 ml syringe | Ingredient | Amount (mg) |
| | Dimethylsulfoxide | 117 |

Risperidone particle size was characterised by light scattering and provided the following distribution of particle size: d(0.1) = 27.49 $\mu$m, d(0.5) = 79.90 $\mu$m and d(0.9) = 176.66 $\mu$m.

Inherent viscosity of the irradiated polymer, as calculated by the technique described in example 1 was 0.25 dl/g.

The risperidone implantable formulation was prepared by connecting male and female syringes and moving the plungers forwards and backwards upon complete dissolution of the polymer and the formation of a homogeneous suspension of the risperidone in the polymer solution.

**Example 7: Depot formulation with Resomer® 503 irradiated at 15 KGy.**

In this example, a lactic-co-glycolic acid copolymer with 50% content of each of the two organic acid monomers and a molecular weight of 32 KDa was sterilised by beta irradiation at 15 KGy under controlled temperature and humidity conditions. The resultant polymer was characterised by its molecular weight according to the method described in example 1. The molecular weight after the irradiation process was 28.3 KDa.

| Female 2.25 ml syringe | Ingredient | Amount (mg) |
|---|---|---|
| | Lactic-co-glycolic acid copolymer (N-capped) with 50% content of each of the two organic acid monomers and a molecular weight of 32 KDa, beta-irradiated in bulk with a 15 KGy dose achieving a final molecular weight of 28.3 KDa. | 50 |
| | Risperidone | 25 |
| Male 2.25 ml syringe | Ingredient | Amount (mg) |
| | Dimethylsulfoxide | 117 |

Risperidone particle size was characterised by light scattering and provided the following distribution of particle size: d(0.1) = 27.49 $\mu$m, d(0.5) = 79.90 $\mu$m and d(0.9) = 176.66 $\mu$m.

Inherent viscosity of the irradiated polymer, as calculated by the technique described in example 1 was 0.25 dl/g.

The risperidone implantable formulation was prepared by connecting male and female syringes and moving the plungers forwards and backwards upon complete dissolution of the polymer and the formation of a homogeneous suspension of the risperidone in the polymer solution.

**Example 8: Depot formulation with Resomer® 504 without irradiation**

| | Ingredient | Amount (mg) |
|---|---|---|
| In this example, a lactic-co-glycolic acid copolymer with 50% content of each of the two organic acid monomers and a molecular weight (according to method described in example 1) of 48 KDa was used. Female 2.25 ml syringe | Lactic-co-glycolic acid copolymer (N-capped) with 50% content of each of the two organic acid monomers and a molecular weight of 48 KDa. | 50 |
| | Risperidone | 25 |
| Male 2.25 ml syringe | Ingredient | Amount (mg) |
| | Dimethylsulfoxide | 117 |

Risperidone particle size was characterised by light scattering and provided the following distribution of particle size: d(0.1) = 27.49 μm, d(0.5) = 79.90 μm and d(0.9) = 176.66 μm.

Inherent viscosity of the irradiated polymer, as calculated by the technique described in example 1 was 0.33 dl/g.

The risperidone implantable formulation was prepared by connecting male and female syringes and moving the plungers forwards and backwards upon complete dissolution of the polymer and the formation of a homogeneous suspension of the risperidone in the polymer solution.

### Example 9: Depot formulation with Resomer® 504 irradiated at 25 KGy.

The current example demonstrates the concept is also valid to achieve an intramuscularly injectable risperidone formulation suitable to be administered once each 4 weeks.

A lactic-co-glycolic acid copolymer with 50% content of each of the two organic acid monomers and a molecular weight of 50 KDa was sterilised by beta irradiation at 25 KGy under controlled temperature and humidity conditions. The resultant polymer was characterised by its molecular weight according to the method described in example 1. The molecular weight after the irradiation process was 35 KDa.

| | Ingredient | Amount (mg) |
|---|---|---|
| Female 2.25 ml syringe | Lactic-co-glycolic acid copolymer (N-capped) with 50% content of each of the two organic acid monomers and a molecular weight of 50 KDa, beta-irradiated in bulk with a 25 KGy dose achieving a final molecular weight of 35 KDa. | 50 |
| | Risperidone | 25 |
| | Dimethylsulfoxide | 117 |

Risperidone particle size was characterised by light scattering and provided the following distribution of particle size: d(0.1) = 17.41 μm, d(0.5) = 51.61 μm and d(0.9) = 175.32 μm.

Inherent viscosity of the irradiated polymer, as calculated by the technique described in example 1 was 0.28 dl/g.

The risperidone implantable formulation was prepared by connecting male and female syringes and moving the plungers forwards and backwards upon complete dissolution of the polymer and the formation of a homogeneous suspension of the risperidone in the polymer solution.

### Example 10: Clinical evaluation of prolonged-release injectable composition

This was a phase III multicentre, randomised, double-blind, placebo controlled clinical trial, which was conducted in the United States and Ukraine, in accordance with the Declaration of Helsinki, and Good Clinical Practice principles outlined in the International Conference on Harmonisation. The protocol, amendments, and informed consent were approved by the Ethics Committee for each site, and written informed consent was obtained from all subjects before study participation.

The study consisted of a screening period of up to 8 days, immediately preceding the baseline day, followed by a treatment period of 12 weeks, which ended with a 2-week follow-up period. Eligible patients were randomly assigned 1:1:1 to double-blind intramuscular treatment with 75 mg or 100 mg of risperidone, in the risperidone LAI depot composition, or placebo. After initial dosing at baseline, each study drug was administered intramuscularly once every 4 weeks during the 12-week treatment period. Risperidone, in the risperidone LAI depot composition (Laboratorios Farmacéuticos ROVI, S.A., Madrid, Spain) was available in a 2-syringe kit , one containing risperidone plus poly lactic-co-glycolic acid (PLGA) in the form of a solid powder, and the other containing dimethyl sulfoxide, the solvent required for reconstitution. They were prepared according to Example 11. Matching placebo was also available in a 2-syringe kit, with a similar appearance but containing only PLGA in the solid potency syringe. Eligible patients were randomised 1:1:1 in a double-blind fashion to risperidone, in the risperidone LAI depot composition, 75 mg, risperidone, in the risperidone LAI depot composition ,100 mg or placebo, injected into the gluteal or deltoid muscle every 4 weeks on days 1, 29, and 57. A unique randomisation number was assigned via Interactive Web Response System (IWRS) accessed immediately after eligibility confirmation of a patient. The doses selected for this study were supported by the results obtained from previously conducted studies, as well as pharmacokinetic modelling. Patients who had never taken risperidone had a brief trial of oral risperidone 2 mg/day for 3 days during the screening period to ensure lack of any hypersensitivity reactions before the first dose of study drug.

Safety was evaluated by assessment of AEs, vital signs, laboratory test, electrocardiograms, physical examinations, ISRs (redness, swelling, and induration), and scales to assess injection site pain (VAS) and extrapyramidal symptoms (AIMS, BARS, and SAS) as well as suicidality (CSSRS).

**Example 11: LAI depot compositions (single unit dose kits)**

*75 mg dose*

A 75 mg dose of the prolonged-release injectable composition comprises the following ingredients in the amounts indicated.

| | Ingredient | Amount (mg) |
|---|---|---|
| Syringe A | PLGA (50:50) having an inherent viscosity of 0.49-0.50 dl/g* | 150 |
| | Risperidone | 75 |
| Syringe B | Dimethylsulfoxide | 350 |
| * Suitable compositions are made with PLGA (50:50) having an inherent viscosity in the range of 0.20-0.60 dl/g, about 0.30-0.55 dl/g, about 0.36-0.52 dl/g, about 0.40-0.58 dl/g, or about 0.46-0.51 dl/g. | | |

*100 mg dose*

A 100 mg dose of the prolonged-release injectable composition comprises the following ingredients in the amounts indicated.

| | Ingredient | Amount (mg) |
|---|---|---|
| Syringe A | PLGA (50:50) having an inherent viscosity of 0.48 to 0.50 dl/g | 200 |
| | Risperidone | 100 |
| Syringe B | Dimethylsulfoxide | 466.7 |
| * Suitable compositions are made with PLGA (50:50) having an inherent viscosity in the range of 0.20-0.60 dl/g, about 0.30-0.55 dl/g, about 0.36-0.52 dl/g, about 0.40-0.58 dl/g, or about 0.46-0.51 dl/g. | | |

**Claims**

1. A prolonged-release injectable composition comprising approximately 75 mg of risperidone, DMSO and PLGA copolymer for use in treating a disease, disorder or condition responsive to risperidone in a subject also undergoing therapy with a drug that is a CYP2D6 (cytochrome P450 2D6) enzyme inhibitor, wherein the use comprises administering said composition to said subject intramuscularly.

2. A prolonged-release injectable composition comprising approximately 75 mg of risperidone, DMSO and PLGA copolymer for use in treating both a disease, disorder or condition responsive to risperidone or senstive to paliperidone in a subject and a disease, disorder or condition responsive to CYP2D6 enzyme inhibitor in the subject, where the use comprises:

    a) administering to said subject a dose of a prolonged-release injectable composition comprising approximately 75 mg of risperidone, DMSO and PLGA copolymer via intramuscular injection; and
    b) within 2 to 4 weeks of said administration, administering to said subject a dose of said CYP2D6 enzyme inhibitor.

3. The composition for use according to any of claims 1 or 2, wherein the use comprises administering at least one dose of said prolonged-release injectable composition at least about every four weeks, and maintaining the dose of said prolonged-release injectable composition substantially constant during a treatment period after administration of said CYP2D6 enzyme inhibitor.

4. The composition for use according to claim 3, wherein the CYP2D6 enzyme inhibitor is a selective serotonin reuptake inhibitor (SSRI).

5. The composition for use according to claim 4, wherein said subject is undergoing treatment with said SSRI for one or more diseases or disorders selected from depression, panic attacks, obsessive-compulsive disorder (OCD), a severe form of premenstrual syndrome (premenstrual dysphoric disorder), anxiety disorders (social and/or general anxiety), post-traumatic stress disorder, bulimia nervosa, major depressive disorder, panic disorder, depressive disorders associated with bipolar I disorder, or treatment-resistant depression (depression that has not improved with at least two other antidepressant treatments).

6. The composition for use according to claim 5, wherein said SSRI is paroxetine or a pharmaceutically acceptable salt thereof or fluoxetine or a pharmaceutically acceptable salt thereof.

7. A prolonged-release injectable composition comprising a dose of about 75 mg of risperidone for use in switching treatment in a subject undergoing treatment with oral risperidone in conjunction with a CYP2D6 enzyme inhibitor with a once-daily oral dosing regimen of risperidone, to a dosing regimen of the prolonged-release injectable composition of risperidone once every approximately 28 days, wherein the use comprises: a) discontinuing once-daily oral administration of risperidone in a subject; and b) within a period of 24-48 hours or within 24 hours from said discontinuation administering to said subject a prolonged-release injectable composition according to any of claims 1 to 6 comprising a 75 mg dose of risperidone, thereby maintaining a therapeutically effective plasma concentration of active moiety in said subject for approximately 28 days.

8. The prolonged-release injectable composition for use according to claim 7, wherein a) when the subject has been receiving once-daily oral doses of less than 4 mg of risperidone, the subject is administered an amount of prolonged-release injectable composition comprising at most 75 mg of risperidone; or b) when the subject has been receiving once-daily oral doses of 4 mg or more of risperidone and/or up to 8 mg of risperidone, the subject is administered an amount of prolonged-release injectable composition comprising no more than 75 mg of risperidone.

9. The prolonged-release injectable composition for use according to claim 8, wherein the dose of CYP2D6 is not altered due to start of treatment with the prolonged-release injectable composition.

10. A prolonged-release injectable composition comprising risperidone, DMSO, and PLGA copolymer for use in treating a disease or disorder responsive to SSRIs and a disease or disorder responsive to risperidone or responsive to paliperidone in a subject in need thereof, wherein the use comprises administering to said subject a therapeutically effective dose of 75 mg of risperidone in said prolonged-release injectable composition and a therapeutically effective dose of SSRI.

11. The prolonged-release injectable composition for use according to claim 10, wherein a) said disease or disorder responsive to SSRIs is selected from the group consisting of depression, panic attacks, obsessive-compulsive disorder (OCD), a severe form of premenstrual syndrome (premenstrual dysphoric disorder), anxiety disorders (social and/or general anxiety), post-traumatic stress disorder, bulimia nervosa, major depressive disorder, panic disorder, depressive disorders associated with bipolar I disorder, or treatment-resistant depression (depression that has not improved with at least two other antidepressant treatments); and b) said disease or disorder responsive to risperidone or paliperidone is selected from the group consisting of psychosis, delusional psychosis, psychotic depression, obsessive-compulsion disorder, schizophrenia, bipolar disorder, schizoaffective disorders, non-schizophrenic psychoses, Asperger's syndrome, Tourette's syndrome, obsessive-compulsive disorder, post-traumatic stress disorder, attention deficit hyperactivity disorder, personality disorders, aggression, depression, dementia, intellectual disability and behavioural disturbances in mental retardation and autism, autistic spectrum disorders, anxiety, eating disorders, anxiety nervous, insomnia, idiopathic dystonia, substance abuse, and any combination thereof.

12. The prolonged-release injectable composition for use according to claim 11, wherein said disease or disorder responsive to risperidone or paliperidone is selected from the group consisting of schizophrenia, schizoaffective disorder, bipolar disorder, and bipolar mania.

13. The prolonged-release injectable composition for use according to any of the preceding claims, wherein: a) said prolonged-release injectable composition is administered intramuscularly once every 28 days or about once monthly; b) More than 0% wt and ≤20% wt of said risperidone is dissolved in said composition prior to administration; c) the PLGA copolymer has a monomer ratio of lactic acid to glycolic acid in the range of about 50:50 to about 75:25; d) the prolonged-release injectable composition has a viscosity in the range of about 0.5-7 Pa·s; e) the prolonged-release injectable composition has a mass ratio of DMSO to risperidone of about 5:1 to about 4:1; and f) the prolonged-release injectable composition has a mass ratio of risperidone to (PLGA +risperidone), expressed as the percentage of the weight of risperidone with respect to the total weight of the risperidone plus PLGA, in the range of about 25-35% .

14. The prolonged-release injectable composition for use according to any of the preceding claims, wherein said use a) excludes the step of administering one or more (plural) loading doses of risperidone in a prolonged-release injectable composition before said intramuscular administration of the prolonged-release injectable composition; and/or b) excludes the step of orally administering one or more doses of risperidone within said 28-day or monthly period.

15. The prolonged-release injectable composition for use according to any of the preceding claims, wherein the subject a) is unstable and experiencing severe to moderate psychotic symptoms; b) is experiencing a first acute exacerbation of schizophrenia; c) is undergoing treatment with one or more oral anti-psychotic drugs; d) has experienced prior episode(s) of acute exacerbation of schizophrenia; e) is experiencing worsening psychotic symptoms or impending relapse of psychosis; f) is experiencing a relapse of severe to moderate psychotic symptoms; and/or g) is undergoing treatment with a prolonged-release injectable composition not according to the invention.

16. The prolonged-release injectable composition for use according to any of the preceding claims, wherein a) the prolonged-release injectable composition comprises a maintenance dose of about 75 mg of risperidone; b) the prolonged-release injectable composition comprises risperidone, DMSO and PLGA copolymer; c) the prolonged-release injectable composition forms a biodegradable implant in the muscle after administration; d) ≤ 2.5%, ≤ 5%, ≤ 7.5%, ≤ 10%, ≤20% of the risperidone dissolves in said composition prior to administration; e) >0%, ≥0.5%, ≥ 1%, ≥ 5%, ≥ 10%, ≥ 15%, or up to about 20% wt of the risperidone is dissolved in said prolonged-release injectable composition prior to administration; f) the PLGA copolymer has a monomer ratio of lactic acid to glycolic acid in the range from about 50:50 to about 75:25, about 35:65 to about 75:25, about 45:55 to about 70:30, about 50:50 to about 65:35, or about 65:35 to about 75:25, 45:55 to 55:45, or 48:52 to 52:48, or about 50:50, i.e. 50:50 ± 10% , or 75:25 ±10%; g) prior to administration, a polymer solution used to form the prolonged-release injectable composition has a viscosity in the range of about 0.5-7 Pa·s, about 0.5-4 Pa·s, about 0.7-4 Pa·s, about 0.5-3.0 Pa·s, about 0.7-3.0 Pa·s, about 1.5-2.1 Pa·s ±10%, about 1.5 to about 2.5 Pa·s, about 1.5 to about 2.3 Pa·s, or about 1.7 to 1.8 Pa·s ± 10%; h) the PLGA copolymer has an inherent viscosity in the range of 0.20-0.60 dl/g, about 0.30-0.55 dl/g, about 0.36-0.52 dl/g, about 0.40-0.58 dl/g, or about 0.46-0.51 dl /g measured in chloroform at 30°C and at a concentration of 0.5% wt with a size 0B Ubbelohde glass capillary viscometer; i) the prolonged-release injectable composition has a mass ratio of DMSO to risperidone of around 5:1 to around 4:1, around 4.6:1 to around 4.8:1, around 4.6:1 to around 4.7:1, around 4.67:1, about 4.66:1 or about 4.68:1 or about 4.66:1; j) the depot composition has a mass ratio of risperidone to (PLGA + risperidone), expressed as the percentage of the weight of risperidone with respect to the total weight of the risperidone plus PLGA, in the range of about 15-40 % wt, about 25-35 % wt, about 30-35 %, about 31-35 %, about 32-34 % or about 33 % wt; k) the PLGA copolymer is end-capped with an ester group or a carboxyl group; l) the content of risperidone in the formulation is about 10-15% wt, about 11-14% wt, about 12-14% wt or about 13% wt; m) the risperidone is partially dissolved or substantially completely undissolved in said composition; n) the PLGA polymer has been irradiated with beta or gamma radiation preferably in the range of 10-30 KGy, more preferably in the range of 10-30 KGy, more preferably in the range of 10-30 KGy, most preferably in the range of 10-30 KGy, most preferably in the range of 10-30 KGy; n) the PLGA polymer has been irradiated with beta or gamma radiation preferably in the range of 10-30 KGy, most preferably in the range of 15-30 Kgy, and most preferably between 16-25 Kgy ±10%; o) the composition is sterile; p) the injectable composition continuously provides therapeutically effective plasma levels of the total active fraction in the patient over a dosing period of at least four weeks from the day of administration; q) prior to administration, the prolonged-release injectable composition has a viscosity in the range of about 1.0-7.0 Pa·s, about 1.5-7.0 Pa·s, or about 1.8-6.5 Pa·s; r) the solvent mass ratio (DMSO) a polymer solution, expressed as a weight percent of solvent with respect to the weight of polymer + solvent, is about 50-75%, about 65-75%, about 60-70%, about 68-72%, or about 70%; s) the concentration of PLGA in the prolonged-release injectable composition is in the range of 24%-50% wt, 24%-40% wt, 24%-30% wt, 25-27% wt, or 26% wt (expressed as the weight percent of polymer based on the total weight of the composition); and/or t) the DMSO content in the prolonged-release injectable composition is about 55-65% wt, about 57-63% wt, about 60-62% wt, or about 61% wt based on the total weight of the composition.

17. The prolonged-release injectable composition for use according to any one of the preceding claims, wherein after said administration, the prolonged-release injectable composition provides a plasma concentration profile of active moiety a) showing a maximum during the initial six days or initial three days or initial two days or initial one to two days of the dosing period; b) showing a maximum during the last 10 to 24 days of a 4-week dosing period; c) showing a maximum during the initial days of the dosing period and a maximum during the remaining days of the dosing period; d) that is substantially level (a standard deviation within $\pm30\%$, $\pm25\%$, $\pm20\%$, $\pm15\%$, $\pm10\%$ or $\pm5\%$ of the mean or average) during the dosing period; e) showing a maximum during the initial 48 hours or initial 72 hours or initial 24 to 48 hours of the dosing period; and/or f) showing a maximum during the last 10 to 28 days or the last 18 to 25 days of a 4- to 5-week dosing period.

18. The prolonged-release injectable composition for use according to any one of the preceding claims, comprising a) providing a container comprising DMSO and a container comprising risperidone and said PLGA copolymer, and mixing the contents of said containers to form said injectable depot composition, then administering said injectable depot composition; or b) providing a container comprising DMSO, a container comprising risperidone, and a container comprising said PLGA copolymer, and mixing the contents of the containers to form said injectable depot composition, then administering said injectable depot composition.

19. The prolonged-release injectable composition for use according to claim 18, wherein said containers are included in a kit.

20. The prolonged-release injectable composition for use according to claim 19, wherein said kit comprises a single dose of risperidone.

**FIG. 1**

**FIG. 2A**

**FIG. 2B**

# INTERNATIONAL SEARCH REPORT

| | International application No. |
|---|---|
| | PCT/ES2023/070320 |

## A. CLASSIFICATION OF SUBJECT MATTER

See extra sheet

According to International Patent Classification (IPC) or to both national classification and IPC

## B. FIELDS SEARCHED

Minimum documentation searched (classification system followed by classification symbols)
A61K, A61P

Documentation searched other than minimum documentation to the extent that such documents are included in the fields searched

Electronic data base consulted during the international search (name of data base and, where practicable, search terms used)

EPODOC, INVENES, WPI, EMBASE, BIOSIS, MEDLINE, NPL, XPESP

## C. DOCUMENTS CONSIDERED TO BE RELEVANT

| Category* | Citation of document, with indication, where appropriate, of the relevant passages | Relevant to claim No. |
|---|---|---|
| X | OKEDI-Autorización of comercialización conforme al Reglamento (CE) n° 726/2004 del Parlamento Europeo and del Consejo al medicamento para uso humano "OKEDI - risperidona" (EU/1/21/1621); 14.02.2022. Retrieved from <URL: Okedi, INN-risperidone (europa.eu)> | 1-20 |
| A | US 2015150791 A1 (GUTIERRO ADURIZ IBON *et al.*) 04/06/2015; the whole document. | 1-20 |
| A | US 2003144220 A1 (OBACH R SCOTT) 31/07/2003; paragraphs [0001], [0018], [0019], [0029], [0033]-[0037], [0043], [0044], [0046]), [0052], [0056]. | 1-20 |

☐ Further documents are listed in the continuation of Box C.   ☒ See patent family annex.

| * Special categories of cited documents: | "T" | later document published after the international filing date or priority date and not in conflict with the application but cited to understand the principle or theory underlying the invention |
|---|---|---|
| "A" document defining the general state of the art which is not considered to be of particular relevance. | | |
| "E" earlier document but published on or after the international filing date | | |
| "L" document which may throw doubts on priority claim(s) or which is cited to establish the publication date of another citation or other special reason (as specified) | "X" | document of particular relevance; the claimed invention cannot be considered novel or cannot be considered to involve an inventive step when the document is taken alone |
| "O" document referring to an oral disclosure use, exhibition, or other means. | "Y" | document of particular relevance; the claimed invention cannot be considered to involve an inventive step when the document is combined with one or more other documents , such combination being obvious to a person skilled in the art |
| "P" document published prior to the international filing date but later than the priority date claimed | "&" | document member of the same patent family |

| Date of the actual completion of the international search | Date of mailing of the international search report |
|---|---|
| 17/10/2023 | **(17/10/2023)** |

| Name and mailing address of the ISA/ | Authorized officer |
|---|---|
| OFICINA ESPAÑOLA DE PATENTES Y MARCAS | N. Vera Gutierrez |
| Paseo de la Castellana, 75 - 28071 Madrid (España) | |
| Facsimile No.: 91 349 53 04 | Telephone No. 913495544 |

Form PCT/ISA/210 (second sheet) (July 2022)

INTERNATIONAL SEARCH REPORT

Information on patent family members

| International application No. |
| PCT/ES2023/070320 |

| Patent document cited in the search report | Publication date | Patent family member(s) | Publication date |
|---|---|---|---|
| US2015150791 A1 | 04.06.2015 | RS64451 B1 | 29.09.2023 |
| | | CY1124783T T1 | 25.11.2022 |
| | | CY1124369T T1 | 22.07.2022 |
| | | PL2582395T T3 | 21.08.2023 |
| | | PT2582395T T | 18.08.2023 |
| | | LT2582395T T | 10.08.2023 |
| | | DK2582395T T3 | 07.08.2023 |
| | | FI2582395T T3 | 08.08.2023 |
| | | US2022280417 A1 | 08.09.2022 |
| | | US11752094 B2 | 12.09.2023 |
| | | US2022192972 A1 | 23.06.2022 |
| | | US11752093 B2 | 12.09.2023 |
| | | US2022133623 A1 | 05.05.2022 |
| | | US11759416 B2 | 19.09.2023 |
| | | HUE057236T T2 | 28.04.2022 |
| | | HRP20211853T T1 | 04.03.2022 |
| | | SI2394663T T1 | 28.02.2022 |
| | | ES2897976T T3 | 03.03.2022 |
| | | PL2394663T T3 | 21.02.2022 |
| | | US2022062164 A1 | 03.03.2022 |
| | | LT2394663T T | 10.02.2022 |
| | | MY174999 A | 01.06.2020 |
| | | PT2394663T T | 26.11.2021 |
| | | ES2878112T T3 | 18.11.2021 |
| | | HUE054922T T2 | 28.10.2021 |
| | | PL2529756T T3 | 15.11.2021 |
| | | HRP20211057T T1 | 01.10.2021 |
| | | SI2529756T T1 | 30.09.2021 |
| | | LT2529756T T | 26.07.2021 |
| | | RS62059 B1 | 30.07.2021 |
| | | PT2529756T T | 28.07.2021 |
| | | DK2529756T T3 | 02.08.2021 |
| | | US2021169778 A1 | 10.06.2021 |
| | | US2021077380 A1 | 18.03.2021 |
| | | US11752092 B2 | 12.09.2023 |
| | | UA119430 C2 | 25.06.2019 |
| | | BR112012030686 A2 | 25.08.2020 |
| | | BR112012030686 B1 | 01.11.2022 |
| | | US2020085728 A1 | 19.03.2020 |
| | | US11241377 B2 | 08.02.2022 |
| | | US2019365643 A1 | 05.12.2019 |
| | | US10912735 B2 | 09.02.2021 |
| | | PL2529757T T3 | 30.04.2014 |
| | | US2019328654 A1 | 31.10.2019 |
| | | US11013683 B2 | 25.05.2021 |
| | | US2019321286 A1 | 24.10.2019 |
| | | US11007139 B2 | 18.05.2021 |
| | | US2019254960 A1 | 22.08.2019 |
| | | US2021267883 A9 | 02.09.2021 |
| | | US2019231682 A1 | 01.08.2019 |

Form PCT/ISA/210 (patent family annex) (July 2022)

## INTERNATIONAL SEARCH REPORT

Information on patent family members

International application No.

PCT/ES2023/070320

| Patent document cited in the search report | Publication date | Patent family member(s) | Publication date |
|---|---|---|---|
| | | US10933015 B2 | 02.03.2021 |
| | | US2019151230 A1 | 23.05.2019 |
| | | US10881605 B2 | 05.01.2021 |
| | | US2019117554 A1 | 25.04.2019 |
| | | US10463607 B2 | 05.11.2019 |
| | | US2018318208 A1 | 08.11.2018 |
| | | US10195138 B2 | 05.02.2019 |
| | | US2018221272 A1 | 09.08.2018 |
| | | US10182982 B2 | 22.01.2019 |
| | | ME02501 B | 20.02.2017 |
| | | BR112014029209 A2 | 27.06.2017 |
| | | ZA201409299 B | 23.12.2015 |
| | | CY1117964T T1 | 17.05.2017 |
| | | CY1117969T T1 | 17.05.2017 |
| | | MY161930 A | 15.05.2017 |
| | | HUE029056T T2 | 28.02.2017 |
| | | PT2575890T T | 06.09.2016 |
| | | PT2394664T T | 06.09.2016 |
| | | HUE029895T T2 | 28.04.2017 |
| | | RS55190 B1 | 31.01.2017 |
| | | LT2575890T T | 26.09.2016 |
| | | LT2394664T T | 26.09.2016 |
| | | NZ703321 A | 27.01.2017 |
| | | HRP20161049T T1 | 30.12.2016 |
| | | PL2575890T T3 | 30.12.2016 |
| | | PL2394664T T3 | 30.12.2016 |
| | | ES2592527T T3 | 30.11.2016 |
| | | CL2014003216 A1 | 10.07.2015 |
| | | SMT201600321 B | 10.11.2016 |
| | | SMT201600320 B | 10.11.2016 |
| | | ES2589106T T3 | 10.11.2016 |
| | | HRP20161100T T1 | 04.11.2016 |
| | | SI2575890T T1 | 28.10.2016 |
| | | SI2394664T T1 | 28.10.2016 |
| | | BR112012030707 A2 | 01.11.2016 |
| | | DK2575890T T3 | 12.09.2016 |
| | | DK2394664T T3 | 12.09.2016 |
| | | ZA201209346 B | 28.08.2013 |
| | | ZA201209394 B | 28.08.2013 |
| | | MA37664 A1 | 31.03.2016 |
| | | MA37664 B1 | 30.11.2016 |
| | | IN10672DELNP2014A | 28.08.2015 |
| | | SG11201407961W A | 30.12.2014 |
| | | UA108885 C2 | 25.06.2015 |
| | | JP2015518036 A | 25.06.2015 |
| | | JP6367795B B2 | 01.08.2018 |
| | | US2015196485 A1 | 16.07.2015 |
| | | US10285936 B2 | 14.05.2019 |
| | | US10335366 B2 | 02.07.2019 |

Form PCT/ISA/210 (patent family annex) (July 2022)

# INTERNATIONAL SEARCH REPORT

Information on patent family members

International application No.

PCT/ES2023/070320

| Patent document cited in the search report | Publication date | Patent family member(s) | Publication date |
|---|---|---|---|
| | | MX2014014483 A | 24.02.2015 |
| | | US2015147398 A1 | 28.05.2015 |
| | | US10350159 B2 | 16.07.2019 |
| | | PH12014502667 A1 | 02.02.2015 |
| | | PH12014502667 B1 | 02.02.2015 |
| | | EA201401346 A1 | 30.04.2015 |
| | | EA031819 B1 | 28.02.2019 |
| | | CO7160108 A2 | 15.01.2015 |
| | | KR20150016964 A | 13.02.2015 |
| | | KR101880716B B1 | 17.08.2018 |
| | | CN104363923 A | 18.02.2015 |
| | | NZ604342 A | 28.11.2014 |
| | | IL235849 B | 31.03.2019 |
| | | AU2013269547 A1 | 22.01.2015 |
| | | AU2013269547B B2 | 15.03.2018 |
| | | CA2874765 A1 | 05.12.2013 |
| | | CA2874765 C | 02.06.2020 |
| | | CL2012003350 A1 | 18.10.2013 |
| | | RS53205 B | 30.06.2014 |
| | | SI2529757T T1 | 30.05.2014 |
| | | SMT201400044 B | 07.05.2014 |
| | | ES2456917T T3 | 24.04.2014 |
| | | HRP20140158T T1 | 25.04.2014 |
| | | PT2529757E E | 27.02.2014 |
| | | DK2529757T T3 | 24.02.2014 |
| | | KR20130118742 A | 30.10.2013 |
| | | KR101784330B B1 | 11.10.2017 |
| | | EA201201570 A1 | 30.09.2013 |
| | | EA024211 B1 | 31.08.2016 |
| | | JP2013528612 A | 11.07.2013 |
| | | JP5882993B B2 | 09.03.2016 |
| | | MA34296 B1 | 01.06.2013 |
| | | JP2013527213 A | 27.06.2013 |
| | | JP5923493B B2 | 24.05.2016 |
| | | US2013177603 A1 | 11.07.2013 |
| | | US10058504 B2 | 28.08.2018 |
| | | US2013171202 A1 | 04.07.2013 |
| | | US10085936 B2 | 02.10.2018 |
| | | EP2854858 A1 | 08.04.2015 |
| | | WO2013178812 A1 | 05.12.2013 |
| | | MX2012013937 A | 11.02.2013 |
| | | MX338373 B | 13.04.2016 |
| | | MX2012013867 A | 24.01.2013 |
| | | MX346167 B | 08.03.2017 |
| | | EA201201569 A1 | 30.04.2013 |
| | | EA024155 B1 | 31.08.2016 |
| | | SG185775 A1 | 30.01.2013 |
| | | CN103002917 A | 27.03.2013 |
| | | CN103002917B B | 27.04.2016 |

Form PCT/ISA/210 (patent family annex) (July 2022)

## INTERNATIONAL SEARCH REPORT
Information on patent family members

International application No.

PCT/ES2023/070320

| Patent document cited in the search report | Publication date | Patent family member(s) | Publication date |
|---|---|---|---|
| | | IL223129 A | 29.02.2016 |
| | | AP3524 A | 11.01.2016 |
| | | CN102933234 A | 13.02.2013 |
| | | CA2800641 A1 | 08.12.2011 |
| | | CA2800641 C | 22.05.2018 |
| | | CA2800111 A1 | 08.12.2011 |
| | | CA2800111 C | 22.05.2018 |
| | | AU2011260318 A1 | 10.01.2013 |
| | | AU2011260318B B2 | 27.11.2014 |
| | | EP2529757 A1 | 05.12.2012 |
| | | EP2529757 B1 | 08.01.2014 |
| | | EP2529756 A2 | 05.12.2012 |
| | | EP2529756 A3 | 07.08.2013 |
| | | EP2575890 A1 | 10.04.2013 |
| | | EP2575890 B1 | 01.06.2016 |
| | | EP2582395 A2 | 24.04.2013 |
| | | EP2582395 B1 | 10.05.2023 |
| | | WO2011151356 A2 | 08.12.2011 |
| | | WO2011151356 A3 | 22.03.2012 |
| | | WO2011151355 A1 | 08.12.2011 |
| | | EP2394664 A1 | 14.12.2011 |
| | | EP2394664 B1 | 01.06.2016 |
| | | EP2394663 A1 | 14.12.2011 |
| | | EP2394663 B1 | 13.10.2021 |
| US2003144220 A1 | 31.07.2003 | GEP20043251 B | 25.06.2004 |
| | | MY132882 A | 31.10.2007 |
| | | KR20010104388 A | 24.11.2001 |
| | | TNSN00071 A1 | 10.11.2005 |
| | | YU70101 A | 12.05.2004 |
| | | GT200000041 A | 27.09.2001 |
| | | AR019507 A1 | 20.02.2002 |
| | | GEP20043251 B | 10.12.2003 |
| | | PE20010051 A1 | 06.02.2001 |
| | | PA8493401 A1 | 30.07.2002 |
| | | SV2002000049 A | 02.12.2002 |
| | | ZA200108158 B | 24.07.2003 |
| | | UY26092 A1 | 31.10.2000 |
| | | US2004028755 A1 | 12.02.2004 |
| | | US2004018253 A1 | 29.01.2004 |
| | | TR200102876T T2 | 21.12.2006 |
| | | SK13832001 A3 | 08.01.2004 |
| | | PL359022 A1 | 23.08.2004 |
| | | OA11858 A | 02.03.2006 |
| | | NZ514466 A | 29.10.2004 |
| | | NO20014858L L | 05.12.2001 |
| | | MA26728 A1 | 20.12.2004 |
| | | JP2003523936 A | 12.08.2003 |
| | | JP3704290B B2 | 12.10.2005 |

## INTERNATIONAL SEARCH REPORT

Information on patent family members

International application No.

PCT/ES2023/070320

| Patent document cited in the search report | Publication date | Patent family member(s) | Publication date |
|---|---|---|---|
| | | IS6083 A | 25.09.2001 |
| | | ID30355 A | 22.11.2001 |
| | | WO0059486 A2 | 12.10.2000 |
| | | WO0059486 A8 | 25.07.2002 |
| | | HU0300535 A2 | 28.07.2003 |
| | | HRP20010722 A2 | 31.08.2002 |
| | | EP1242058 A1 | 25.09.2002 |
| | | EE200100524 A | 16.12.2002 |
| | | EA200100934 A1 | 29.08.2002 |
| | | EA005158 B1 | 30.12.2004 |
| | | DZ3032 A1 | 27.03.2004 |
| | | CZ20013599 A3 | 15.01.2003 |
| | | CN1479628 A | 03.03.2004 |
| | | CA2367052 A1 | 12.10.2000 |
| | | BR0009564 A | 08.01.2002 |
| | | BG106075 A | 28.06.2002 |
| | | AU3185000 A | 23.10.2000 |
| | | AU774923B B2 | 15.07.2004 |

Form PCT/ISA/210 (patent family annex) (July 2022)

## INTERNATIONAL SEARCH REPORT

International application No.

PCT/ES2023/070320

CLASSIFICATION OF SUBJECT MATTER

*A61K31/59* (2006.01)
*A61K47/20* (2006.01)
*A61K47/34* (2017.01)
*A61K9/10* (2006.01)
*A61P25/18* (2006.01)

Form PCT/ISA/210 (extra sheet) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 8221778 B, Siegel
- WO 2005070332 A
- US 5688801 A
- US 6803055 B
- US 5770231 A
- US 7118763 B
- US 4389330 A, Dunn
- US 4530840 A
- US 6673767 B, Brodebeck
- US 6143314 A, Chandrashekar
- WO 2004081196 A
- WO 2001035929 A
- WO 2008153611 A2
- WO 2000024374 A
- WO 2002038185 A
- WO 2008100576 A
- WO 2011151355 A1, Rovi, S.A.
- WO 201142453 A
- US 10085936 B, Gutierro Aduriz
- US 10463607 B, Gutierro Aduriz
- US 10182982 B, Gutierro Aduriz
- US 20200085728 A1, Gutierro Aduriz
- EP 2394664 A1, ROVI, S.A.
- US 10058504 B, Gutierro Aduriz
- US 10881605 B, Gutierro Aduriz
- US 10195138 B, Gutierro Aduriz,
- US 20210077380 A1, ROVI, S.A.
- EP 2394663 A1, ROVI, S.A.
- WO 2011151356 A2, ROVI, S.A.
- US 10350159 B, Gutierro Aduriz
- US 20190328654 A1, ROVI, S.A.
- EP 2529757 A1, ROVI, S.A.
- WO 2013178811 A1, ROVI, S.A.
- US 10335366 B, Gutierro Aduriz
- US 20190254960 A1, ROVI, S.A.,
- US 11007139 B, Gutierro Aduriz
- EP 2529756 A2, ROVI, S.A.
- WO 2013178812 A1, ROVI, S.A.
- US 20080287464 A1, Wright
- US 20090264491 A1, McKay
- US 20040010224 A1, Bodmeier
- US 20070077304 A1, Luk
- US 20100015195 A1, Jain
- US 20100266655 A1, Dadey
- WO 9529664 A, Alkermes
- WO 2004011054 A2, Alza
- WO 2007041410 A2, Luk
- WO 2008059058 A1, Bourges
- WO 2010018159 A1, Schoenhammer
- US 6596316 B
- US 6379703 B
- US 6194006 B
- WO 200040221 A
- US 9180197 B
- US 9186413 B
- US 9597402 B
- US 10010612 B
- US 10058554 B
- US 10376590 B
- US 10406160 B
- US 6331311 B, Brodbeck
- US 4938763 A, Dunn
- US 20040266791 A

### Non-patent literature cited in the description

- *Clinical Schizo. Related Psych.*, 2018, 130-141
- **BAI et al.** *Pharmacopsych.*, 2006, vol. 39, 135-141
- **ANDORN et al.** *J. Clin. Psychopharm.*, 2019, vol. 39 (5), 428-433
- **NASSER et al.** Efficacy, safety and tolerability of RBP-7000 once-monthly risperidone for the treatment of acute schizophrenia: an 8-week, randomized, double-blind, placebo-controlled, multicenter Phase 3 study. *J. Clin. Psycopharm.*, 2016, vol. 36 (2), 130-140
- **CORRELL et al.** *NPJ Schizophrenia*, 25 November 2020, vol. 6, 37
- Doria Phase III Trial hits primary endpoint. Edison Investment Research Limited. Laboratorios Farmaceuticos ROVI, S.A., 19 March 2019
- **ANTA et al.** Newer formulations of risperidone: remarks about Risperidone ISM. *CNS Drugs*, 05 September 2020
- **IUPAC.** Basic definitions of terms relating to polymers 1974. *Pure Appl. Chem.*, 1974, vol. 40, 477-491